# EUROPEAN PATENT APPLICATION

(11) **EP 3 842 065 A1**
(43) Date of publication of application: **30.06.2021**
(21) Application number: 19306751.9
(22) Date of filing: 23.12.2019
(51) Int. Cl.: A61K 39/00, C12N 7/02, C12N 15/863, G16B 15/20

(54) **PROCESS FOR DESIGNING A RECOMBINANT POXVIRUS FOR A THERAPEUTIC VACCINE**

(71) Applicant: TRANSGENE, 67400 Illkirch Graffenstaden (FR)
(72) Inventor: Grellier, Benoît, 68440 Habsheim (FR)
(74) Representative: Regimbeau

(57) **Abstract**

The present invention generally relates to a process for designing a recombinant poxvirus for a therapeutic vaccine, i.e. personalized cancer vaccine, said recombinant poxvirus comprising one or more expression cassettes, each for expression of a fusion of a plurality of peptides, i.e. neopeptides, characterized in that it comprises performing the steps of :
(a) selecting a first subset of candidate peptides, wherein said peptides present transmembrane scores below a TMS threshold;
(b) determining an optimal distribution of the candidate peptides from said first subset to the expression cassette(s) among a plurality of possible distributions;
(c) for each expression cassette, determining an optimal slot allocation of the candidate peptides as function of cassette slot occupancy rule;
(d) determining a DNA transfer sequence comprising the nucleotide sequence of the one or more expression cassette(s) for generation of said recombinant poxvirus.

## Description

### TECHNICAL FIELD OF THE INVENTION

The present invention generally relates to a process for designing a recombinant poxvirus comprising one or more expression cassette(s), each for expression of a fusion of a plurality of peptides, a process for preparing such a recombinant poxvirus, as well as such a recombinant poxvirus and its use for treating or preventing a disease, especially for treating or preventing cancer.

### BACKGROUND

During the last decades, numerous therapeutic vaccines expressing antigens have been generated with the goal of stimulating innate and specific immune responses against such antigens. For example, many of the first cancer vaccines were built to prime the immune system against the most commonly identified tumor-associated antigens (TAAs) (e.g. MUC-1, WT1, PSA, CEA) that are overexpressed in the tumor. However, these TAAs can have residual expression in non-tumor cells; thus, efficacy of this traditional approach is expected to be limited by self-tolerance against such "self" antigens. Other groups have also focused on the use of multiepitope polypeptides to improve the efficacy of their candidate vaccine and induce broadly specific T cell responses against various disease-associated antigens (e.g. Depla et al., 2008, J. Virol. 82(1): 435-450). The technology to identify CTL epitopes, synthetize genes encoding multiple multiepitope-containing polypeptides and their delivery through DNA plasmid and viral vectors is now well suited.

Moreover, the traditional paradigm to deliver the same set of cancer antigens to everyone in the population ignores individual variability in disease risk and immunologic responses. Importantly, it could not be ignored that humans respond differently to vaccines and the host's immune response significantly varies in a population (Relman, 2008, J Infect Dis. 198(1):4-5; Plotkin, 2008, Clin Infect Dis.47(3):401-9). With the recent introduction of immune checkpoint blockers in the clinic, it has indeed become clear to the medical staff that some treatments are effective for some patients but not for others.

Advances in immunology, genetics, molecular biology and bioinformatics have paved the way to more personalized approaches. Technological breakthroughs in the field of genome sequencing (e.g. Next Generation Sequencing (NGS)) has now made it possible to sequence the entire genome or exome (coding regions of the genome) of tumor at an unprecedented speed and cost. Molecular characterization of tumors demonstrated that during the process of carcinogenesis and proliferation of cancer cells, mutations occur as a consequence of high rates of proliferations, deficient repair mechanisms and clonal selection. Accumulation of mutations in the tumor genome generally leads to expression of aberrant protein species that are specific to cancerous tissue. Those are referred to as neoantigens. In contrast to the most common tumor-associated antigens, tumor neoantigens are solely present in tumor cells but not in normal cells and do not induce deletion of their antigen-specific T cells in the thymus. Thus, it is expected that they induce strong immune responses without bearing the risk for self-tolerance and autoimmune reactions against self-proteins. Therefore, tumor neoantigens may be ideal targets for designing therapeutic vaccines specifically adapted to the tumor although their adoption in routine care requires overcoming a number of scientific and technical challenges. In particular, most cancer mutations are the result of stochastic phenomenon and are specific to each patient.

Among other topics, successful translation is subordinated to the achievement of an effective manufacturing process to ensure prompt delivery of clinically sufficient amounts to the bedside of the patient because identification of the tumor mutations, design of the neopeptides incorporating such mutations, manufacturing and testing of the personalized vaccine is concomitant to disease progression.

Thus, it has been proposed in the application WO2018/234506 a personalized cancer vaccine comprising a recombinant poxvirus encoding neopeptides (each comprising at least one tumor-specific mutation), and a method for preparing said personalized cancer vaccine. Poxviruses are double-stranded DNA viruses which life cycle takes place in the cytoplasm and uses the cellular machinery and its own viral proteins to replicate.

In more details, this method comprises a step of identifying the neopeptides suitable for being encoded by a so-called personalized cancer vaccine, and a step of generating said recombinant poxvirus. To this end, said peptide-encoding nucleic acid molecules to be inserted in the genome of the recombinant poxvirus are arranged at random but respecting a balance in term of fusion length in one or more "expression cassettes" (preferably 3 cassettes, each encoding a fusion bearing up to 10 peptides) under the control of suitable regulatory elements permitting expression in the subject. The generation of the recombinant poxvirus itself is typically performed through homologous recombination between a parental poxvirus and a "transfer" plasmid comprising the peptide-expression cassettes as well as extra features such as flanking recombination arms and enzyme restriction sites required for the cloning of the expression cassettes. Selection of recombinant poxviruses having integrated the peptide-expression cassettes may be facilitated by the use of reporter gene encoding a selection or colored marker aimed at reflecting insertion of the expression cassettes within the poxviral genome although finer analysis (such as PCR) are required to confirm the recombinant status.

However, it has been suspected that expression of hydrophobic peptides and peptides that bear transmembrane (TM) domains, might impair the generation or production of the recombinant virus, thus disabling the development of some vaccine candidates. When the TM domain is within a given peptide (intra peptide TM), said peptide may simply be discarded, but it has also been observed that a TM domain may be generated by the junction of two particular peptides (inter peptide TM) within a fusion encoded by an expression cassette, which requires to modify the order of the peptides. Another characteristic of poxviruses is their sensitivity to homologous recombination events. Under control, it is an advantage for genetic engineering, but remains an issue when unexpected homologous regions are incorporated.

A simple way to deal with this problem is to simply remove TMs and peptides with high hydrophobicity index, but this is not very effective as illustrated in Example 5 of WO2018/234506. This example shows how tricky is the generation of recombinant MVA designed to express a set of 30 peptides distributed in three expression cassettes. Suppression of interpeptide (by peptide inversion) and intrapeptide (by elimination of the TM-containing peptides) potential TM segments did not permit to generate expressing recombinant MVA. Nor did the reduction of hydrophobicity scoring of the peptide fusion (from 133 to 28.5 scores). This illustrates that even when selecting peptides free of TMs and peptides with low hydrophobicity index, the generation or production of recombinant poxvirus stays very low.

There is consequently a need for a solution to design optimized expression cassettes, for expression of a plurality of peptides or one or more peptide fusion(s). The present invention proposes a process based on the following properties: hydrophobicity and hydrophobic-related protein features, propensity to form transmembrane domains and sequence homology; and on the position of the peptides of one over the other inside the fusion. As demonstrated in the Example section, the process of the invention permits to increase the yield of recombinant poxvirus generation.

This technical problem is solved by the provision of the embodiments as defined in the claims.

Other and further aspects, features and advantages of the present invention will be apparent from the following description of the presently preferred embodiments of the invention. These embodiments are given for the purpose of disclosure.

### BRIEF SUMMARY OF THE INVENTION

According to a first aspect, the present invention is related to a process for designing a recombinant poxvirus, said recombinant poxvirus comprising one or more expression cassettes, each for expression of a fusion of one or a plurality of peptides, characterized in that it comprises performing the steps of:
(a) selecting a first subset of candidate peptides, wherein said peptides present transmembrane scores below a TMS threshold;
(b) determining an optimal distribution of the candidate peptides from said first subset to the expression cassette(s) among a plurality of possible distributions;
(c) for each expression cassette, determining an optimal slot assignment of the candidate peptides as function of cassette slot occupancy rule;
(d) determining a DNA transfer sequence comprising the nucleotide sequence of the one or more expression cassette(s) for generation of said recombinant poxvirus.

According to advantageous and non-limiting features:
Said peptides of step (a) present continuous regions below a homology threshold.

Step (a) comprises discarding peptides if they present transmembrane scores above a TMS threshold and/or an homologous region with a higher ranked candidate above an homology threshold, and selecting a second subset of the identified set of candidate peptides as candidate peptides neither disclosed nor selected in the first subset.

Step (b) comprises, if at least one fusion peptide presents an hydropathy score above a given threshold, replacing the candidate peptide from the first subset of candidate peptides presenting the highest hydropathy score by a candidate peptide from the second subset, and proceeding again with a random peptide selection.

Said optimal distribution of step (b) presents the lowest range between the hydropathy scores of at least two fusion peptides.

Step (c) further comprises discarding any fusion peptide that displays at least one TM patch with a score above a TMSK7 threshold.

step (c) further comprises the selection of the peptide fusion with the lowest TM score.

Step (c) comprises, if at least one transmembrane domain is detected in any possible slot assignment of the candidate peptides in an expression cassette, replacing the candidate peptide from the first subset of candidate peptides presenting the highest transmembrane score, by a candidate peptide from the second subset, and repeating steps (b) then (c).

Said cassette slot occupancy rule defines possible slot positions of candidate peptides within a cassette according to transmembrane scores of the peptides, and, in case of equal transmembrane scores, according to their hydropathy scores.

The candidates peptides distributed to an expression cassette are classified according to at least three classes of risk of not generating or producing any recombinant poxvirus, wherein said cassette slot occupancy rule defining, for each slot positions of a cassette, what class a candidate peptide shall have to be assigned to this slot position.

Said optimal slot assignment of the candidate peptides distributed to an expression cassette presents a transmembrane score below a given threshold.

There is/are a single expression cassette if the number of selected candidate peptides is below 10, two expression cassettes if the number of selected candidate peptides is between 10 and 14, and three expression cassettes if the number of selected candidate peptides is between 15 and 30.

According to a second aspect, the invention is related to a process for preparing a therapeutic vaccine comprising a recombinant poxvirus, comprising:
- performing the process according to the first aspect for designing said recombinant poxvirus;
- generating said recombinant poxvirus.

According to advantageous and non-limiting features:
Said process further comprises a manufacturing step of said recombinant poxvirus, wherein said manufacturing step comprises an amplification step in a suitable producer cell to a suitable scale, a step of recovery of the produced recombinant poxvirus from the cell culture and an optional step of purification of the recovered recombinant poxvirus.

Said recombinant poxvirus encodes neopeptides and is for use as a personalized cancer vaccine.

According to a third aspect, the invention is related to a personalized cancer vaccine obtained according to the process according to the second aspect, wherein said personalized cancer vaccine is a composition comprising a therapeutically effective amount of said recombinant poxvirus and a pharmaceutically acceptable vehicle, preferably for use in a subject in need thereof for treating a cancer or preventing its relapse in a subject.

### BRIEF DESCRIPTION OF THE DRAWINGS

**Figure 1** represents the general scheme of a process according to the invention for designing a recombinant poxvirus for expression of fusion(s) of a plurality of peptides. The main steps (a) to (d) are indicated in grey boxes (HSK7: fusion hydropathy score; HSK7Thresh: HSK7 threshold; TM patch: transmembrane patch; TMSK7: fusion TMS threshold).
**Figure 2** represents an architecture for performing the process according to the invention involving a server 1, data processing means 11, storage means 12 and an interface 13.
**Figure 3** is a flowchart illustrating an embodiment of a step (a) of selecting the candidate peptides suitable for expression by the recombinant poxvirus in the process according to the invention (TMS: transmembrane score; TMSThresh: peptide TMS threshold; N: number of total candidate peptides; Nmax: maximal number of peptides).
**Figure 4** is a flowchart illustrating an embodiment of a step (b) of determining an optimal inter-cassette distribution of the candidate peptides in the process according to the invention (L: low; M: medium; H: high). The classes L, M and H refer to the risk of not generating or producing any recombinant poxvirus (TMS: transmembrane score; HS: hydropathy score ; HSK7: fusion hydropathy score; HSK7Thresh: HSK7 threshold; Nmin: minimal number of peptides; R: range; RThresh: range threshold; iter: iteration).
**Figure 5** is an example of table for distributing peptides to cassettes according to the total number of peptides and the classes of peptides in the inter-cassette distribution (L: low; M: medium; H: high).
**Figure 6** is an example of ranking of peptides according to their transmembrane scores and hydropathy scores (TMS: transmembrane score; HS: hydropathy score; L: low; M: medium; H: high).
**Figure 7** is a flowchart illustrating an embodiment of a step (c) of determining for each expression cassette an optimal slot assignment of the candidate peptides (TMS: transmembrane score ; TM patch: transmembrane patch; TMSK7Thresh: fusion TMS threshold; iter: iteration).
**Figure 8** represents an example of slot occupancy rule depending on the number of peptides;
   A) diagram representing an embodiment of occupancy rule of 10 slots depending on the number n of peptides of the cassette and on the peptide's classes (light grey: "low" class; medium grey: "medium" class; dark grey: "high" class);
   B) table of slot occupancy according to the number n of peptides of the cassette and the peptide's classes, showing the number of possible combinations.

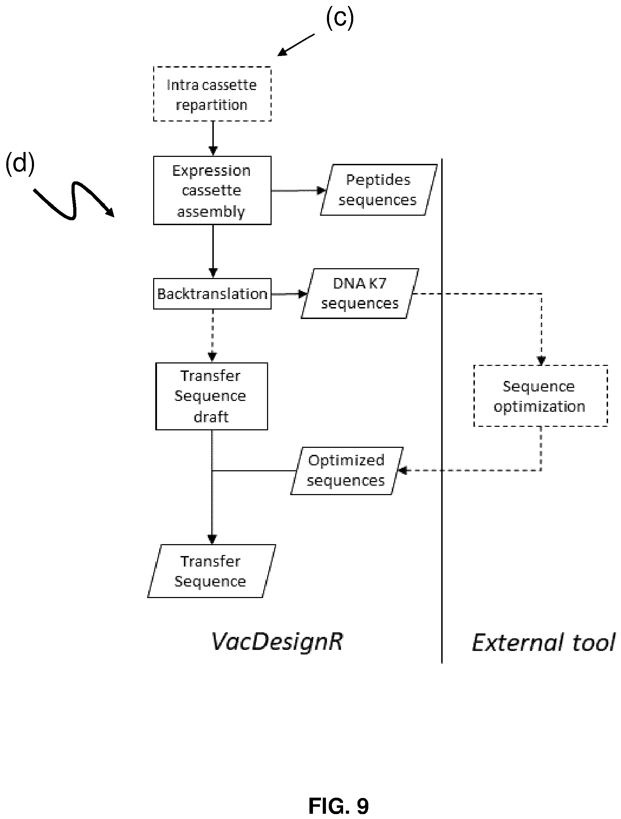

**Figure 9** is a flowchart illustrating an embodiment of a step (d) of back translation according to the invention, allowing the determination of a DNA transfer sequence to be inserted in a recombinant poxvirus.

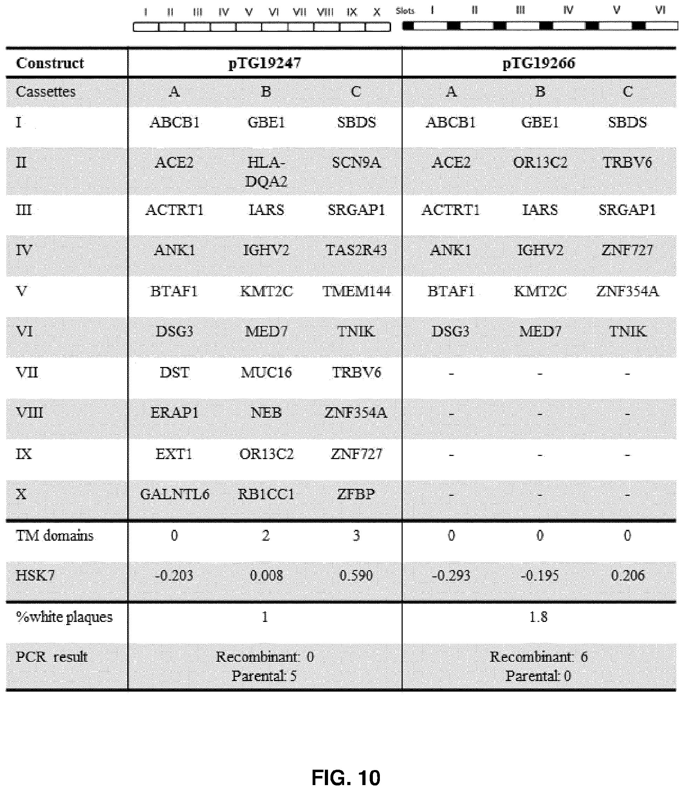

**Figure 10** shows examples of recombinant poxviruses bearing three expression cassettes (A, B and C) (pTG19247 and pTG19266 expressing respectively 10 and 6 neopeptides) obtained using a manual approach for designing a personalized cancer vaccine (TM domains: transmembrane domains; HSK7: fusion hydropathy score; PCR: polymerase chain reaction).

### DETAILED DESCRIPTION OF THE INVENTION

### General definitions

Unless defined otherwise, all technical and scientific terms used herein have the same meaning as commonly understood by one of ordinary skill in the art to which the invention pertains.

The term "a" and "an" refers to "one" or to "more than one" of the grammatical object of the article (i.e., at least one including 2, 3, 4, 5, etc.) unless the context clearly dictates otherwise.

The term "and/or" wherever used herein includes the meaning of "and", "or" and "all or any other combination of the elements connected by said term".

The terms "such as", "e.g.", as used herein are for illustrative purposes and thus non-limiting.

The term "about" or "approximately" is used herein to indicate that value or range given herein is not critical and can vary within 10%, preferably within 8%, and more preferably within 5% of the given value or range so as to include the inherent variation of error for a device or a method being employed to determine such a value or range, or the variation that exists among the tested subjects.

As used herein, when used to define products, compositions and methods, the term "comprising" (and any form of comprising, such as "comprise" and "comprises"), "having" (and any form of having, such as "have" and "has"), "including" (and any form of including, such as "includes" and "include") or "containing" (and any form of containing, such as "contains" and "contain") are open-ended and do not exclude additional, unrecited elements or method steps. "Consisting essentially of" means excluding other components or steps of any essential significance. "Consisting of" means excluding more than trace elements of other components or steps.

The terms "polypeptide", "peptide" and "protein" are used interchangeably to refer to polymers of amino acid residues covalently linked by peptide bonds. The polymer can be linear, branched or cyclic and may comprise naturally occurring and/or amino acid analogs and it may be interrupted by non-amino acids. No limitation is placed on the maximum number of amino acids. As a general indication, the term peptide preferably refers to short polymers (e.g. comprising at least 9 amino acid residues) whereas polypeptide or protein designates longer polymers (typically comprising more than 50 amino acids). These terms encompass native polymers, modified polymers (also designated derivatives, analogs, variants or mutants) as well as fragments thereof. In the context of the present invention, a polypeptide may also be in the form of a fusion of various peptides as well as a multimer (e.g. dimers), among others. In the context of the present invention, the term peptide as used herein also encompasses neopeptide.

The term "neopeptide" refers to a peptide comprising at least a tumor-specific mutation as described herein.

The term "fusion" as used herein refers to the combination of two or more peptides as a single polypeptide chain.

Within the context of the present invention, the terms "nucleic acid", "nucleic acid molecule", "polynucleotide", "nucleic acid sequence" and "nucleotide sequence" are used interchangeably and define a polymer of at least 9 nucleotide residues in either deoxyribonucleic acid (DNA) or ribonucleic acid (RNA) or mixed polyribopolydeoxyribonucleotides. These terms encompass single or double-stranded, linear or circular, natural or synthetic, unmodified or modified versions thereof (e.g. genetically modified polynucleotides, optimized polynucleotides), sense or antisense polynucleotides, chimeric mixture (e.g. RNA-DNA hybrids). Exemplary DNA nucleic acids include without limitation, complementary DNA (cDNA), genomic DNA, plasmid DNA, vectors, viral DNA (e.g. viral genomes, viral vectors), oligonucleotides, probes, primers, coding DNA, non-coding DNA, or any fragment thereof, etc. Exemplary RNA nucleic acids include, without limitation, messenger RNA (mRNA), precursor messenger RNA (pre-mRNA), coding RNA, non-coding RNA, etc. Nucleic acid sequences described herein may be synthesized by standard methods known in the art, e.g. by use of an automated DNA synthesizer (such as those that are commercially available from Biosearch, Applied Biosystems, etc.) or obtained from a naturally occurring source (e.g. a genome, cDNA, etc.) or an artificial source (such as a commercially available library, a plasmid, etc.) using molecular biology techniques well known in the art (e.g. cloning, PCR, etc.).

In a general manner, the term "identity" refers to an amino acid to amino acid or nucleotide to nucleotide correspondence between two polypeptides or nucleic acid sequences. The percentage of identity between two sequences is a function of the number of identical positions shared by the sequences, taking into account the number of gaps which need to be introduced for optimal alignment and the length of each gap. Various computer programs and mathematical algorithms are available in the art to determine the percentage of identity between amino acid sequences, such as for example the Blast program available at NCBI or ALIGN in Atlas of Protein Sequence and Structure (Dayhoffed, 1981, Suppl., 3: 482-9). Programs for determining identity between nucleotide sequences are also available in specialized data base (e.g. Genbank, the Wisconsin Sequence Analysis Package, BESTFIT, FASTA and GAP programs).

The terms "virus", "viral particle", "viral vector" and "virion" are used interchangeably and are to be understood broadly as meaning a vehicle comprising at least one element of a wild-type virus genome that may be packaged into a viral particle. The term encompasses the viral genome and the virus particles.

As used herein the term "poxvirus" refers to a virus belonging to the *Poxviridae* family.

The term "recombinant" relates to genetically engineering. When used in connection with a virus, and notably with the poxvirus described herein, it indicates that the virus has been engineered to contain inserted in its genome at least one exogenous nucleic acid molecule (also called recombinant gene or nucleic acid), that is not found in or expressed by a naturally-occurring virus genome. Nevertheless, the exogenous nucleic acid can be homologous or heterologous to the subject into which the recombinant virus is introduced. Advantageously in the context of the invention, said exogenous nucleic acid is one or more expression cassette, each encoding a plurality of peptides preferably arranged in a fusion.

The term "obtained from", "originating" or "originate" is used to identify the original source of a component (e.g. a (neo)epitope, (neo)peptide, (neo)antigen, a nucleic acid molecule, a virus, etc.) or the original source of a sample (e.g. a subject or a group of subjects) but is not meant to limit the method by which the component/sample is made which can be, for example, by chemical synthesis or recombinant means.

As used herein, the term "isolated" refers to a component (e.g. a polypeptide, nucleic acid molecule, vector, etc.), that is removed from its natural environment (i.e. separated from at least one other component(s) with which it is naturally associated or found in nature). More specifically, it refers to a component that is purified (partially or substantially). For example, a nucleic acid molecule is isolated when it is separated of sequences normally associated with it in nature (e.g. dissociated from a chromosome or a genome) but it can be associated with heterologous sequences (e.g. within a recombinant vector). A synthetic component is isolated by nature.

The term "subject" generally refers to a vertebrate organism for whom any of the product or methods disclosed herein is needed or may be beneficial. Typically, the organism is a mammal, particularly a mammal selected from the group consisting of domestic animals, farm animals, sport animals, and primates (human and non-human). The terms "subject" and "patient" may be used interchangeably when referring to a human organism and covers male and female as well as a fetus, newborn, infant, young adult, adult and elderly.

The term "administering" (or any form of administration such as "administered", etc.) as used herein refers to the delivery to a subject of a component (e.g. a recombinant poxvirus) according to the modalities described herein.

### Design of recombinant poxvirus

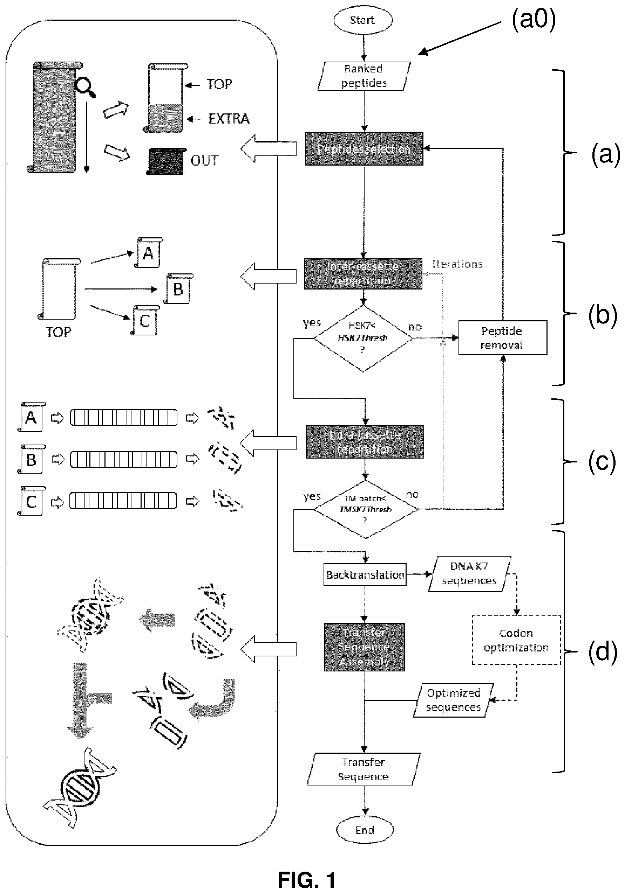

In a first aspect, the present invention relates to a process for designing a recombinant poxvirus, said recombinant poxvirus comprising one or more expression cassette(s), each for expression of a plurality of peptides, and preferably a fusion of a plurality of peptides. The general scheme of this process is illustrated by **Figure 1****.**

By "designing" the recombinant poxvirus, it is here in particular meant determining an identified set of "candidate" peptides, their distribution in one or more expression cassettes and their slot assignment in a peptide fusion, and to determine a DNA transfer sequence, so that the recombinant poxvirus having such a DNA transfer sequence is suitable for use as a therapeutic vaccine, notably for treating infectious diseases or proliferative diseases such as cancers. Preferably, the process of the present invention comprises at least 4 steps, (a) a step of selecting a first subset of peptides suitable for being expressed by the recombinant poxvirus (i.e. the candidate peptides) that are selected from a peptide list on the basis of different criteria as described herein, (b) a step of inter-cassette distribution (e.g. distribution of the candidate peptides to one or more expression cassette(s)); (c) a step of intra-cassette slot assignment (e.g. determining for each expression cassette an optimal assignment of the candidate peptides distributed to the expression cassette, in other words ordering the candidate peptides within the expression cassette) and (d) a step of determining a DNA transfer sequence comprising the nucleotide sequence of the one or more expression cassette(s) for the generation of the recombinant poxvirus. In other words, the process of the invention permits to select candidate peptides from a list of peptides, to elaborate peptide fusion and repartition within one or more expression cassette(s) and generate expression cassette's nucleotide sequence in accordance with poxvirus specifications.

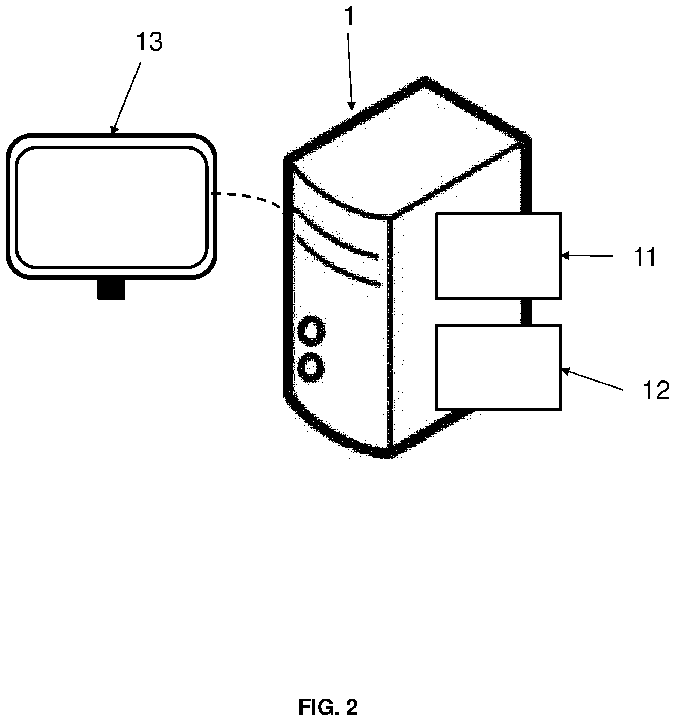

With reference to **Figure 2**, the present designing of said recombinant poxvirus is intended to be performed by data processing means 11 (for instance a processor) of a server 1. Said server 1 may further comprise data storage means 12 (i.e. a memory) notably for storing peptides databases, and an interface 13 (i.e. screen, mouse, keyboard, connectors with further devices, etc.).

### Poxvirus

In one embodiment, the recombinant poxvirus to be generated by the designing process of the invention is obtained from the *Chordopoxvirinae* subfamily directed to vertebrate host which includes several genus such as *Orthopoxvirus*, *Capripoxvirus*, *Avipoxvirus*, *Parapoxvirus*, *Leporipoxvirus* and *Suipoxvirus.* In a preferred embodiment, the recombinant poxvirus for use herein is generated from a poxvirus belonging to the *Orthopoxvirus* genus and even more preferably to the vaccinia virus (VV) species. Any vaccinia virus strain can be used in the context of the present invention including, without limitation, Western Reserve (WR), Copenhagen (Cop), Lister, LIVP, Wyeth, Tashkent, Tian Tan, Brighton, Ankara, MVA (Modified vaccinia virus Ankara), LC16M8, LC16M0 strains, etc., with a specific preference for WR, Copenhagen, Wyeth and MVA vaccinia virus. Sequences of the genome of various Poxviridae, are available in the art in specialized databanks such as Genbank (e.g. accession numbers NC_006998, M35027 and U94848 provide sequences of WR, Copenhagen, and MVA genomes). In another embodiment, the recombinant poxvirus for use herein can be generated from a Parapoxvirus, with a particular preference for one of a pseudocowpox virus (PCPV) species. PCPV possesses a genome which is a linear and double-stranded segment of DNA, typically of 130-150 kilobases.

Appropriate poxvirus for use herein are described in WO2018/234506. In the context of the present invention, one may use either a wild-type strain as well as any derivative thereof (i.e. a poxvirus that is modified compared to the wild-type strain, e.g. by truncation, deletion, substitution, and/or insertion of one or more nucleotide(s) contiguous or not within the viral genome). Exemplary modifications are preferably in viral genes involved in DNA metabolism, host virulence or IFN pathway (see e.g. Guse et al., 2011, Expert Opinion Biol. Ther.11(5):595-608). A particularly suitable gene to be disrupted is the thymidine kinase (TK)-encoding gene (locus J2R; Genbank accession number AAA48082). Alternatively, or in combination with, the poxvirus for use herein may be modified by altering at least one gene or both genes encoding viral ribonucleotide reductase (RR). The viral enzyme is composed of two heterologous subunits, designed R1 and R2 encoded respectively by the I4L and F4L locus. Sequences for the I4L and F4L genes and their locations in the genome of various poxvirus are available in public databases. The gene nomenclature used herein is that of Copenhagen Vaccinia strain. It is also used herein for the homologous genes of other poxviridae unless otherwise indicated and correspondence between Copenhagen and other vaccinia strains is available to the skilled person. For illustrative purposes, vaccinia viruses (VV) defective for TK, or TK and RR are described in the literature (see e.g. WO2009/065546).

Preferably, the recombinant poxvirus to be designed by the designing process of the invention is a replication-defective poxvirus, and preferably a replication defective vaccinia virus, which means that it cannot replicate to any significant extent in human cells.

A particularly appropriate poxvirus for use in the context of the present invention is MVA due to its highly attenuated phenotype (Mayr et al., 1975, Infection 3: 6-14; Sutter and Moss, 1992, Proc. Natl. Acad. Sci. USA 89: 10847-51). The nucleotide sequence of the MVA genome and amino acid sequence of the encoded viral proteins are available in the art, e.g. from Antoine et al. (1998, Virol. 244: 365-96) and Genbank (accession number U94848).

### Plurality of Peptide(s)

The term "plurality" as used herein refers to the state of being numerous (e.g. at least 5 or above). The number of peptides (i.e. the plurality of peptides) that can be encoded by the recombinant poxvirus is not limiting depending on the type of poxvirus selected (e.g. MVA) and poxvirus-mediated expression (e.g. expression in short or large fusions and regulatory elements as described hereinafter). For illustrative purposes, 5 to 150 peptides may be expressed by the recombinant poxvirus, preferably from 7 to 100, more preferably from 9 to 40 and even more preferably 10 to 35 peptides, with a preference for about 30 peptides (e.g. 27, 28, 29, 30, 31, 32 or 33).

The length of the peptides to be selected by the process according to the invention for being encoded by the recombinant poxvirus is typically comprised between 12 amino acid residues to about 150 amino acid residues but of course the length can vary from one peptide to another. For illustrative purposes, each of the peptide(s) may have a length of 13 to 101 amino acid residues, desirably of 16 to 90 amino acid residues, preferably of 17 to 85 amino acid residues, more preferably of 18 to 80 amino acid residues and, even more preferably of 20 to 40 amino acid residues.

### Neopeptides

The process of the invention is particularly adapted to the development of a personalized cancer vaccine and allows to identify and provide a set of candidate peptides comprising at least one tumor-specific mutation on a patient by patient basis, i.e., candidate neopeptides. The neopeptide is thus of non-self-nature (not found in a self-protein) as explained herein. Due to this non-self-nature, it is expected that such peptide(s) will be recognized by the tumor specific T lymphocytes.

In a preferred embodiment, the process of the present invention is carried out for selecting a plurality of neopeptides. Typically, a "neopeptide" is a peptide corresponding to a fragment of a neoantigen and, thus, comprising a minimal immune determinant (i.e., a "neoepitope") that contributes to MHC-dependent T cell recognition (or that is presented by MHC molecules at the surface of cells of the subject), and at least a tumor-specific mutation that is non-silent. Typically, the tumor-specific mutation is located within the neoepitope and is surrounded (on either or both side(s)) by flanking sequence(s) naturally present in its normal environment (which is/are those of the neoantigen from which the neoepitope is taken).

The term "neoantigen" as used herein refers to an antigen that has emerged during the carcinogenesis process in a cancer cell. Thus, a neoantigen is found in cancer cells or tissues obtained from a patient but not found in a sample of normal cells or tissues obtained from a patient or a heathy individual. Typically, a tumor-specific mutation is preferably present in the DNA contained in a cancer cell (e.g. the tumor sample) but absent in the DNA contained in a non-cancerous cell (e.g. the non-tumor sample).

The term "mutation" relates to at least one sequence difference between a test sequence (e.g. neoantigen) and a reference sequence (self-antigen). Several types of tumor-specific mutations are encompassed by the present invention, including missense mutations, deletions, insertions, frameshift mutations, and mutations in splicing site (see WO2018/234506). In the context of the present invention, a tumor-specific mutation is preferably non-silent and translates in a change at the amino acid level with respect to the corresponding self-antigen. More preferably, it is missense or frameshift. A "missense" mutation results from the substitution of one nucleotide with another nucleotide within a specific codon that affects the encoded amino acid sequence, thus resulting in one amino acid change. Another way to affect a protein sequence, is an insertion or a deletion of one or more nucleotide(s) (e.g. a piece of DNA) which changes the number of nucleotides in a nucleic acid molecule. Insertion and deletion mutations may result in change of the reading frame (a so-called frame shift mutation) but not necessarily in case of in-frame indels (e.g. the insertion or deletion of a multiple of 3 nucleotides will result in the addition or suppression of at least one codon). The almost entire amino acid sequence may be changed if the mutation occurs early in the nucleotide sequence. A frameshift mutation may also result in the generation of a stop codon that is translated into a stop signal and the resulting protein will then be truncated (deleted of its portion downstream the *de novo* generated stop codon). A missense mutation may also be located in the splicing sites of the mRNA leading to an aberrant splicing and therefore to aberrant protein sequence.

With reference to the neopeptide embodiment, it is preferred that at least 70% (e.g. 80%, 85%, 90%, 95% and even 100%) of the neopeptides to be selected have a single missense mutation centered in the middle of the neopeptide (e.g. the mutated amino acid is exactly positioned in the middle of the neopeptide (in case the neopeptide has an odd number of amino acids) or at one of the two central position (in case the neopeptide has an even number of amino acids), or at any of the 2 to 5 amino acids on each side of the exact central position, with the same number of flanking amino acids on either side of the mutated amino acid).

The mutation may however, at least in some of the neopeptides, be located close to N-terminus or C-terminus, especially for a frameshift mutation or when the missense mutation occurs at or close to the N or C-terminus of the neoantigen.

Of course, the neopeptides length shares the features described above in connection with the peptide embodiment. Preferably, for illustrative but not limitative purposes, neopeptides comprising a missense mutation have individually a length of 20 to 40, and preferably 25 to 35 amino acid residues. Particular preference is given to individual neopeptides of 25, 27 or 29 residues (preferably the missense mutation is located in position 13 (25mer), 14 (27mer) or 15 (29mer) starting from the N-terminus of the neopeptide flanked by 12 (25mer), 13 (27mer) or 14 (29mer) amino acids on each side of the mutation).

### Fusion of peptides

As explained, the present process contemplates expression by the recombinant poxvirus of a plurality of peptides (e.g. neopeptides) allocated according to the process of the present invention in the form of one or more fusion(s). The position of a peptide in a peptide fusion is called a "slot". The number of peptides may vary from one fusion to another fusion with a preference for a fusion comprising 2 to 15 peptides, preferably, 3 to 12, more preferably 4 to 11, and even more preferably, 5 to 10 peptides (e.g. 5, 6, 7, 8, 9 or 10).

Certain embodiments contemplate the presence of a signal peptide at the N-terminus of the peptide fusion to enhance the processing through ER (endoplasmic reticulum) and/or secretion. Briefly, signal peptides usually comprise 15 to 35 essentially hydrophobic amino acids, are inserted at the N-terminus of the polypeptide downstream of the codon for initiation of translation and are then removed by a specific ER-located endopeptidase to give the mature polypeptide. Appropriate signal peptides are known in the art. They may be obtained from cellular or viral polypeptides such as those of immunoglobulins, tissue plasminogen activator, insulin, rabies glycoprotein, the HIV virus envelope glycoprotein or the measles virus F glycoprotein (gp) or may be synthetic. If more than a signal peptide sequence is to be used in the recombinant poxvirus, one may choose ones of different origin (e.g. from the rabies or the measles F gp) and/or degenerate the homologous sequences that show a high degree of sequence identity (e.g. above 75%) so as to limit homologous recombination events that might deteriorate the production process (see WO2008/138649).

In the context of the present invention, the fusion of peptides can be direct (i.e. without any additional amino acid residues in between) or through a linker to improve accessibility of the peptides. Typically, linkers are composed of a short stretch of amino acid residues such as glycine (Gly or G), serine (Ser or S), threonine (Thr or T), asparagine (Asn or N), alanine (Ala or A) and/or proline (Pro or P). Preferred linkers in the context of this invention comprise 2 to 10 amino acids, with a preference for 3, 5, or 10 amino acids, mainly glycine and serine (e.g. composed of one of more amino acid motifs such as GSG, GST, GAS or GTS). It is within the reach of the skilled person to assess the need to include a linker or not between two fused peptides. In a preferred embodiment, peptides are arranged in fusions with linkers in between each peptide (e.g. between peptide 1 and peptide 2, between peptide 2 and peptide 3, etc.) and, optionally, at the N terminus of the first peptide. The linkers nucleic sequences comprised in one recombinant poxvirus may be degenerated by taking advantage of the codon degeneracy (4 codons available for encoding a G residue, 6 for encoding a S residue, 4 for encoding a T residue etc.), thus contributing to reduce the sequence identity within the recombinant poxvirus and limiting undesirable recombination events that may occur during the production process.

Certain embodiments of the present invention also contemplate the presence of a tag (typically a short peptide sequence able to be recognized by available antisera or compounds) in order to facilitate the detection of the expression of the peptides (or fusion(s) thereof) or of infected host cells expressing such peptides (or fusion thereof). A vast variety of tag peptides can be used in the context of the invention including, without limitation, PK tag, FLAG octapeptide, MYC tag, HIS tag (usually a stretch of 4 to 10 histidine residues) and e-tag (US 6,686,152). The tag peptide(s) may be independently positioned at the N-terminus of the protein or alternatively at its C-terminus or alternatively internally or at any of these positions when several tags are employed. Tag peptides can be detected by immunodetection assays using anti-tag antibodies.

In the context of the present invention, each fusion may be designed differently from the other(s) and may distinguish one another by the presence, and/or the sequence and/or the number and/or the positioning of elements such as peptide signals, linkers, tags, etc. According to a preferred embodiment, each fusion however comprises a) a signal peptide at its N terminus, b) linkers at the N-terminus of the first peptide, between each peptide and at the C-terminus of the last peptide and c) a tag at its C-terminus.

### Expression of the peptide fusion(s)

In accordance with the present invention, each peptide fusion is placed under the control of appropriate regulatory elements (notably a promoter and a termination sequence), referred to as "expression cassette". Typically, an "expression cassette" comprises a nucleic acid molecule encoding one or more peptide(s) (e.g. neopeptide(s)) or peptide fusion, under the control of suitable regulatory elements permitting expression in the subject. In other words, each of the one or more expression cassette encoding a peptide fusion as described herein is equipped with suitable regulatory elements for expression in a host cell or subject. As used herein, the term "regulatory element" or "regulatory sequence" refers to any element that allows, contributes or modulates the expression of a nucleic acid (e.g. encoding the peptide fusion described herein) in a given host cell or subject, including replication, duplication, transcription, splicing, translation, stability and/or transport of the nucleic acid(s) or its derivative (i.e. m RNA).

It will be appreciated by those skilled in the art that the choice of the regulatory elements can depend on such factors as the expression cassette itself, the virus into which it is inserted, the host cell or subject, the level of expression desired, etc. The promoter is of special importance. Poxvirus promoters are particularly adapted for directing expression of a given nucleic acid from a poxvirus such as the recombinant poxvirus described herein. Representative examples include without limitation the vaccinia 7.5K, H5R, 11K7.5 (Erbs et al., 2008, Cancer Gene Ther. 15(1): 18-28), TK, p28, p11, pB2R, pA35R and K1L promoters, as well as synthetic promoters such as those described in Chakrabarti et al. (1997, Biotechniques 23: 1094-7; Hammond et al., 1997, J. Virol Methods 66: 135-8; and Kumar and Boyle, 1990, Virology 179: 151-8) as well as early/late chimeric promoters.

Those skilled in the art will appreciate that further to the promoter, the regulatory elements may further comprise additional elements for proper initiation, regulation and/or termination of transcription (e.g. polyA transcription termination sequences), mRNA transport (e.g. signal sequences as described herein), stability (e.g. introns and non-coding 5' and 3' sequences), translation (e.g. an initiator Met, tripartite leader sequences, IRES ribosome binding sites, signal peptides, etc.) and identification and purification (e.g. tag peptides as described herein).

In a preferred embodiment, all peptides selected by the process of the invention are clustered in 1 to 5 expression cassettes, with a preference for 1 to 3 cassettes, preferably 2 or 3 cassettes, and more preferably 3 cassettes of 2 to 15 peptides. In a particularly preferred embodiment, the recombinant poxvirus comprises three cassettes each encoding a fusion of 5 to 10 peptides with a preference for approximately 10 peptides each. For illustrative purposes, if the recombinant poxvirus comprises 3 expression cassettes, each of them will use different promoters to control the nucleic acid sequence encoding each peptide fusion, for example the pH5R promoter for the first cassette, the pC11R promoter for the second cassette and the p7.5K promoter for the third cassette.

### Step (a) of the designing process: identification and selection of the candidate peptides suitable for expression by the recombinant poxvirus

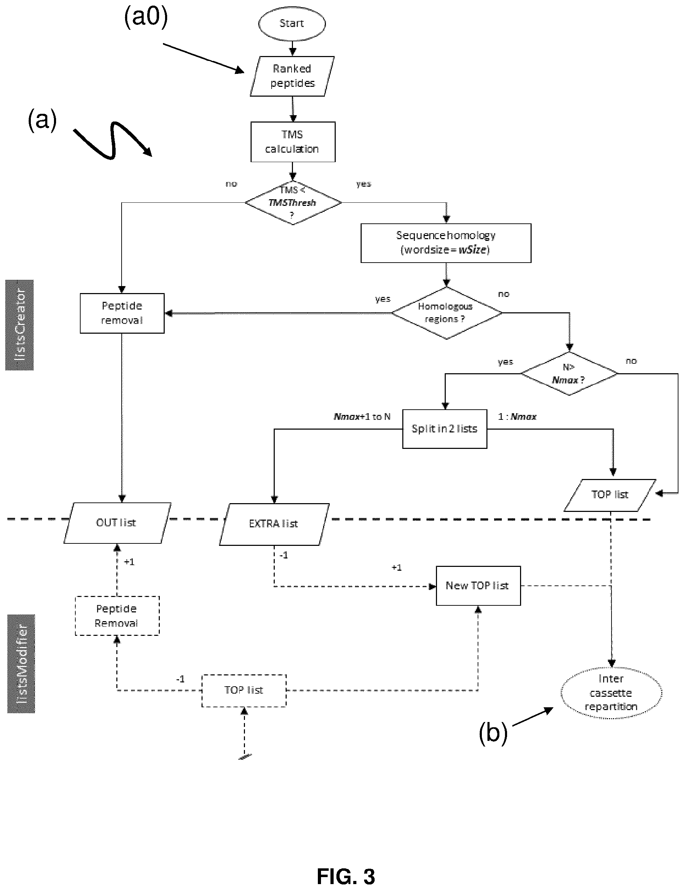

The designing process according to the present invention first requires an identification and ranking of peptides (step (a0)) and a selection of candidate peptides to be expressed by the recombinant poxvirus (step (a)), as illustrated in **Figure 3**.

As explained, the present process for designing a recombinant poxvirus assumes that a set of peptides of interest is available. Step (a0) allows the selection of a set of peptides of interest, via the identification and the ranking of said peptides. Said set of peptides of interest can also be designed by "peptides from the input file". In a preferred embodiment, said peptides of interest are identified and/or ranked according to one or more criteria, e.g. the predicted immunogenic potential of the peptides. For sake of clarity, "immunogenic potential" refers to the capacity of a peptide to raise an immune response once delivered to a subject (e.g. via a recombinant poxvirus encoding such peptide or peptide fusion). In the context of the present invention, the immune response can be a humoral or a T cell response (or both), including a CD4+ (e.g. Th1, Th2 and/or Th17) and/or a CD8+ T cell response (e.g. a CTL response). A large number of prediction algorithms exists in the art for *in silico* prediction of the immunogenic potential of a peptide or a peptide fusion, notably to predict the capacity to raise a T cell response (see e.g. Nielsen et al., 2010, Immunology 130(3): 319-28). Algorithms relying on peptide binding affinity predictions to MHC molecules are appropriate in the context of the present invention. For illustrative purposes, one may cite SVMHC, NetMHCII, Tepitope/propped, syfpeithi, Epitollkit, etc. Note that the number of candidate peptides may be higher than the final number of peptides actually expressed by the cassettes (for example, in order to have 10 to 35 final peptides as proposed, there might be hundreds of candidate peptides).

Step (a) individually assesses said identified and ranked peptides and allows the selection of candidate peptides. By "candidate", it is meant suitable for generating the recombinant poxvirus.

In one embodiment, the selection of candidate peptides is carried out through filtering means based on one or more criteria. In this step, the processing means (11) (**Figure 2**) creates one or more subsets of the set of peptides of interest. These peptides can be discarded and placed in an OUT list or can be considered as suitable for generating the recombinant poxvirus. In the case where the peptides are considered as suitable for generating the recombinant poxvirus, said identified candidate peptides are put in a first subset (TOP list) or in a second subset (EXTRA list), wherein said TOP list contains the maximum number of candidate peptides for generating the recombinant poxvirus with the highest prediction, and wherein said EXTRA list comprises the remaining candidate peptides.

In a preferred embodiment of the invention, the set of peptides of interest, or peptides from the input file, are independently or additionally filtered out based on a criteria corresponding to their propensity to bear a transmembrane (TM) segment. A "TM segment" or "TM domain" as used herein can be defined as being a short hydrophobic alpha helix of approximately 20 amino acid residues. The transmembrane score ("TM score", or "TMS") is indicative of the probability that the peptide presents or forms at least one TM segment(s) within its sequence (i.e. intra TM). The higher the TMS is, the more important the probability that a peptide sequence to present or form a TM domain is. The TM score can be determined using several prediction tools well known to the skilled person, such as TMHMM (for Transmembrane Hidden Markov Model; Krogh et al., 2001, J. Mol. Biol. 305: 567-80), the DAS (Dense Alignment Surface), wherein the DAS-TM filter algorithm provides a high precision hydrophobicity profile for the query from which the location of the potential TM segments can be obtained, or the method based on the relationship between hydrophobicity and hydrophobic moment in the propensity of producing transmembrane domains (Eisenberg et al., 1982, Nature, Sep 23;299(5881):371-4), wherein the correlation between hydrophobicity and hydrophobic moment defines the protein section as globular, transmembrane or superficial.

Typically, the TM scores are calculated based on the amino acid composition of each individual peptide. Preferably, the TM score calculation is based on the relationship between hydrophobicity and hydrophobic moment in the propensity of producing transmembrane segments, as defined by Eisenberg. TMS calculation is preferably performed using the *membpos* function, based on the standardized scale of Eisenberg (Eisenberg et al., 1984, Annual review of biochemistry 53.1: 595-623), using windows of 11 amino acids for calculate the theoretical fragment type. Preferably, said *membpos* function is based on at least two criteria selected from the group comprising the aminoacid sequence and the protein rotational angle. In said aspect of the invention, a discrete value of "1" is attributed to transmembrane, and a discrete value of "0" is attributed to globular or surface. The TM score is then computed by calculating a cumulative sum of the local discrete values, including a "continuity threshold", used to consider spatial location of TM patches in a linear sequence. Increasing the continuity threshold allows the occurrences of "0" discrete values in the cumulative calculation. Said incremental calculation is performed in both directions to avoid any bias. The TM score is therefore the sum of the TM patches. The highest score is used and corresponds to the TM score.

For example, the TMS for the peptide TVHHRIVGCSLAVICGVLYGSTFQVPIIYI was calculated with a continuity threshold of 0 and 1 (Table1). With a continuity threshold of 0, the calculated TM patches were 36_3_1 in both directions, and a TM score of 40. With a continuity threshold of 1, the calculated TM patch was 36_8 (TM score = 44) in one direction, and 36_7 (TM score = 43) in the other direction. The two TM scores being different, the highest is used: for a continuity threshold of 1, the TM score is 44. In this example, by extending the continuity threshold to 3 or more, no local patch is taken into account, and the TMS is higher.

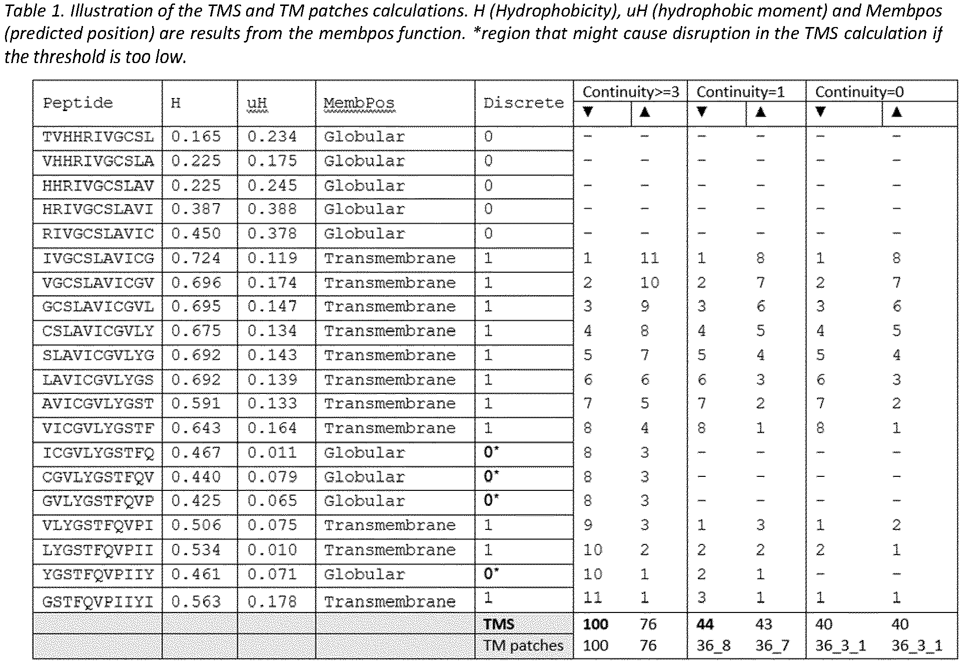

In a preferred embodiment, the process for designing a recombinant poxvirus, comprises selecting a first subset (TOP list) of candidate peptides, wherein said candidate peptides present transmembrane scores below a TMS threshold. Said process further comprises discarding peptides (OUT list) if they present transmembrane scores exceeding TMS thresholds and selecting a second subset (EXTRA list) of the identified set of candidate peptides as candidate peptides neither disclosed nor selected in the first subset.

In a more preferred embodiment, said candidate peptides of the first and second subset present transmembrane scores below a threshold of 40, more preferably a threshold of 30, with a specific preference for a threshold of 25 (e.g. of preferred TM scores : 24, 23, 22, 21, 20, 19, 18, 17, 16, 15, 14, 13, 12, 11, 10, etc.).

For illustrative purposes, peptides of interest having a TM score below a threshold of 25 as determined according to the method described by Eisenberg et al. are kept and placed in the set of candidate peptides, comprising the first and second subset.

In still another and preferred embodiment of the invention, the set of peptides of interest, or peptides from the input file, are independently or additionally filtered out based on a criteria corresponding to the peptides sequences homology in order to limit the recombination events between peptides.

Peptides having sequences bearing continuous regions with more than X identical amino acids are considered homologous, wherein X is 12, more preferably 11, more preferably 10, more preferably 9, more preferably 8, more preferably 7, more preferably 6, and even more preferably 5. Only one peptide among those having such homologous continuous regions is kept, said peptide being selected with the highest rank score (e.g. predicted immunogenic potential). The peptides not kept are discarded and put in the OUT list. For example, in a list of peptides comprising 3 peptides having homologous continuous sequences of 5 or more amino acids, the one having the highest immunogenic potential rank score is kept, wherein the two others are discarded.

Duplication is a specific case of homology. It may occur when the mutation present in a given peptide (e.g. a tumor-specific mutation of a given neopeptide) is part of several epitopes or when they were given a score for several HLA types. Preferably, only one peptide among such duplicated peptides is kept, said peptide being selected with the highest immunogenic potential rank score; the other duplicated peptide(s) are discarded.

In a preferred embodiment, the process for designing a recombinant poxvirus comprises selecting a first subset of candidate peptides, wherein said candidate peptides present continuous regions below a homology threshold. Preferably, peptides of interest presenting continuous regions below a homology threshold of 5 are kept and placed in the set of candidate peptides, comprising the first and second subset.

In a preferred embodiment of the invention, the selection step (a) comprises selecting a first subset of candidate peptides, wherein said peptides present transmembrane scores below a TMS threshold (e.g. below 25). In a more preferred embodiment, said peptides of step (a) present continuous regions below a homology threshold (e.g. below 5). Preferably, the filtering steps are sequentially performed in the following order: 1) TMS threshold; 2) homology threshold (**Figure 3**).

Therefore, the outputs are up to 3 lists (each list still preferably ranked according to their immunogenicity):
1. The OUT list: comprises the discarded peptides;
2. The TOP list: comprises the maximum number of candidate peptides for generating the recombinant poxvirus, said peptides having the highest immunogenic prediction rank scores (the first subset);
3. The EXTRA list: comprises the remaining eligible candidate (the second subset).

Practically, step (a) typically comprises first discarding candidates which present TM scores exceeding the TMS threshold (e.g. candidates having a TM score equal or above the threshold of 25) and/or those having homologous regions (e.g. candidates having more than 5 contiguous amino acids in common with an eligible peptide) and then, from the N remaining candidate peptides, selecting the up to Nmax best ones (wherein Nmax is the maximum number of peptides to be expressed by the recombinant poxvirus, such as about 30) as the first subset and keeping the N-Nmax others as the second subset (**Figure 1** **and** **3**).

However:
- The second subset may be empty (if N<Nmax, thus the first subset only contains N peptides);
- If the number of peptides selected in the first subset is under m peptides (wherein Nmin is the minimum number of peptides for generating a recombinant poxvirus, such as about 5) because transmembrane scores and/or peptides sequence homology of candidate peptides are too high, it is deemed impossible to generate a recombinant poxvirus and the process fails.

### Step (b) of the designing process: inter-cassette distribution

In a preferred embodiment, the process of the invention further comprises a step (b) for determining an optimal distribution of the candidate peptides from said first subset to the one or more expression cassette(s) among a plurality of possible distributions.

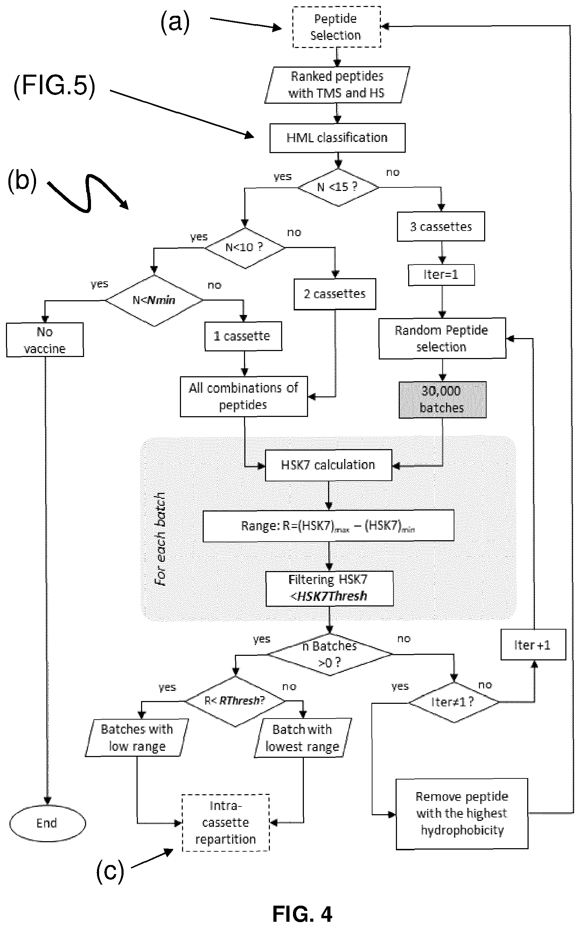

Step (b) allows to balance the quality and quantity of peptides in each expression cassette in order to avoid any bias due to an overrepresentation of peptides that might be "at risk" for the poxvirus generation and production (**Figure 4**).

In one embodiment of step (b), the selected peptides of the first subset (TOP list) generated at step (a) are categorized into different classes, based on the peptides TM scores and peptides hydropathy scores (HS). In particular, the peptides of the TOP list subset are preferably categorized into three classes, "Low" (L), "Medium" (M) and "High" (H). These classes refer to the risk (for each peptide alone) of not generating or producing any recombinant poxvirus.

It is to be understood that the words "low", "medium" and "high" are relative, and just express that the classes allow to compare the peptides of the TOP list subset. In other words, the L peptides (the peptides of the L class) are the most susceptible of generating or producing any recombinant poxvirus, the H peptides (the peptides of the H class) are the least susceptible of generating or producing any recombinant poxvirus, and the M peptides (the peptides of the M class) are less susceptible of generating or producing any recombinant poxvirus than the L peptides, but more susceptible of generating or producing any recombinant poxvirus than the H peptides.

More preferably, the peptides of the TOP list are ranked according to their TM scores (preferably below 25). In the case of equal TM scores, the peptides are ranked according to their hydropathy score. The hydropathy score of a given peptide or peptide fusion is calculated by dividing the hydrophobicity score determined for said peptide or fusion by the number of residues present in said peptide or said fusion. In a general manner, the hydrophobicity score of a given peptide is determined by the sum of the hydrophobicity/hydrophilicity value of each amino acid residue of said peptide, as described for example in WO2018/234506; the hydrophobicity score of a peptide fusion corresponds to the sum of the hydrophobicity scores determined for each peptide comprised in said fusion. Technically, the amino acid sequence of the one or more peptide or fusion peptide thereof encoded by a recombinant poxvirus is hydrophilic in nature. The hydrophilic or hydrophobic nature of a given sequence can be easily determined by a number of methods and algorithms available in the art. It is within the reach of the man skilled in the art to calculate the hydrophobicity score and/or the hydropathy score of a specific sequence. For instance, these scores can be determined using the Kyte-Doolittle method (Kyte and Doolittle, 1982, J. Mol. Biol. 157: 105-32) or any other suitable method (e.g. Rose et al., 1993, Ann. Rev. Biomol. Struc. 22: 381-415; Kallol et al., 2003, J. Chromat. 1000: 637-55; Sweet et al., 1983, J. Mol. Biol. 171: 479-88; among many others) or algorithm (e.g. ExPAsy Prot Scale Protein; Protein Hydrophobicity Plots developed by Colorado state or the World of Bioinformatics, etc.).

As explained above, the levels for determining the classes are relative, there is no particular thresholds involved, and advantageously, the number of peptides of each class (L, M or H) is only a function of the number of peptides N.

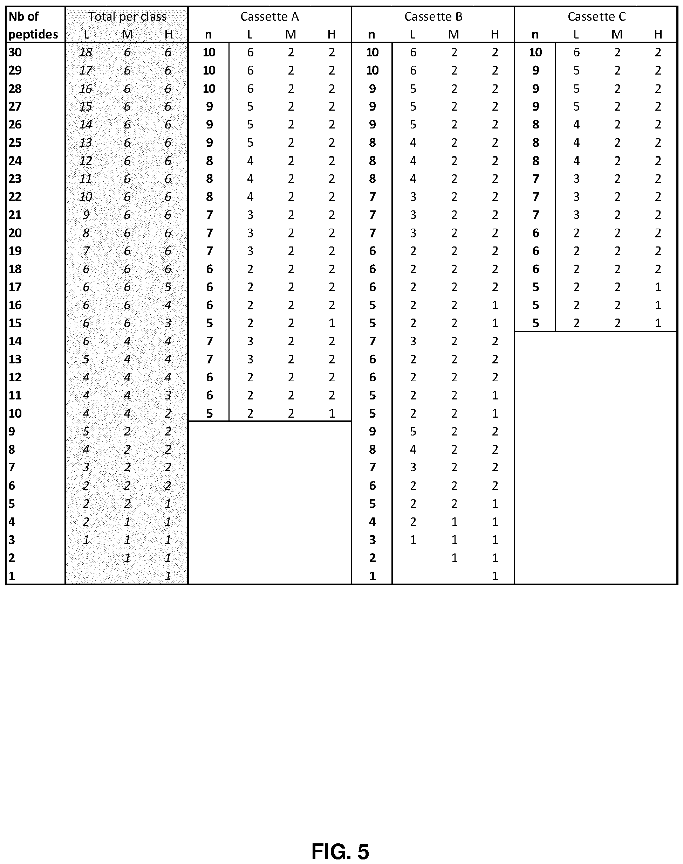

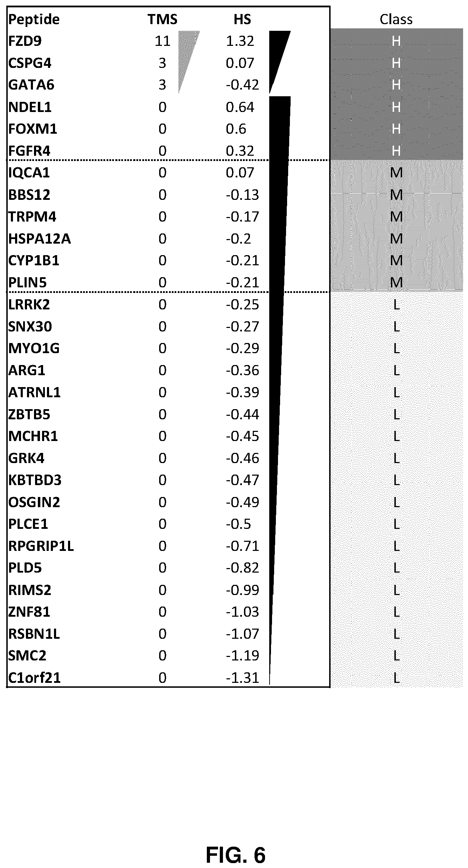

In a preferred embodiment, the number of candidate peptides of each class distributed to each class complies with a first distribution table including, without limitation, the table given in **Figure 5**. In this figure, More specifically, the column "Nb of peptides" indicates the number of peptides each recombinant poxvirus shall contain (here, the number of peptides is comprised between 1 and 30). the column "total per class" shows how many peptides of each class the whole recombinant poxvirus shall express as a function of the number of selected peptides. **Figure 6** gives an example of peptide ranking and determination of their classes. Here, the number of peptides is 30. **Figure 5** indicates that for 30 peptides, there are 6 H, 6 M and 18 L peptides. The 30 peptides are ranked according to their TM scores, and, in case of equal TM scores, according to their hydropathy scores. Here, 3 peptides have a TM score above 0: they are put in the H class. As the 27 other peptides have a TM score of 0, their hydropathy scores are considered for their ranking. For the 27 peptides, the 3 peptides with a HS comprised between 0.32 and 0.64 are put in the H class, the 6 peptides with a HS comprised between -0.21 and 0.07 are put in the M class, and the 18 peptides with a HS comprised between -1.31 and -0.25 are put in the L class.

In a preferred embodiment, step (b) further comprises the determination of an optimal distribution of the candidate peptides from the first subset of categorized peptides to the different expression cassettes ("inter-cassette distribution") among a plurality of possible distributions. By "distribution", it is simply meant to designate for each selected peptide which cassette will encode this peptide, independently from their order within the cassette ("intra-cassette slot assignment"). The distribution preset is based on the number of total candidate peptides (N) of the TOP first subset, and the accepted number of peptides from each class in each cassette.

By "optimal" distribution, it is meant the distribution having the lowest global risk of not generating or producing any recombinant poxvirus, criteria for reaching such optimal distribution will be explained below.

As represented by **Figure 4**, the number of cassettes is determined as a function of the number of selected peptide of the first subset: typically 3 cassettes if the first subset contains at least 15 peptides (at least 5 per cassette), 2 cassettes if the first subset contains 10 to 14 peptides (5 to 7 per cassette), 1 cassette if the first subset contains less than 10 peptides (provided that the minimal number of selected is reached as explained, typically 5 peptides). In case of 1 cassette, the distribution is trivial, and in case of 2 cassettes, there is only a binary choice for each peptide, so that the number of combinations stays low and they can be all tried. In case of 3 cassettes, the number of combinations becomes extremely high, so that the process preferably works through iterations ("iter") of only a given number of batches, i.e. possible combinations (30,000 batches for example, randomly chosen among all possible combinations). If none of these batches is satisfactory, at least a second iteration (i.e. 30,000 further batches) is attempted.

Preferably, the number of candidate peptides distributed to each cassette complies with a distribution table, see for example **Figure 5**. Said table is also advantageously stored by storage means 12 of the server 1. As illustrated, and in accordance with the table of **Figure 5**, if the number of selected peptides is dividable by 3, each cassette shall receive the same number of peptides, else there shall be at maximum one peptide of difference between two cassettes. For example, in case of 26 peptides, 26≥15 so that 3 cassettes are used, with 9 peptides in the first and second cassettes, and 8 peptides in the third cassette.

Further, the table of **Figure 5** defines the number of peptides (n) of each class (L, M or H) each cassette (A, B and C) shall contain as a function of the number of peptides distributed to the cassette. For example, wherein the number of peptides is 13, a first cassette shall express 7 peptides, including 3 L peptides, 2 M peptides, and 2 H peptides; and a second cassette shall express 6 peptides, including 2 L peptides, 2 M peptides, and 2 H peptides. Globally, as presented in the right column, the poxvirus shall express 5 L peptides, 4 M peptides, and 4 H peptides.

In a more preferred embodiment, step (b) further comprises the calculation of the hydropathy score of each fusion peptide (also named "hydropathy score of the content of the expression cassette", or "hydropathy score of the expression cassette") for each batch and the comparison to a fusion hydropathy threshold (HSK7Thresh). Preferably, said hydropathy threshold is equal to or below 0.2, preferably below 0.19, preferably below 0.18, preferably below 0.17, preferably below 0.16, and even more preferably below 0.15.

If at least one expression cassette presents a hydropathy score above said threshold, the corresponding batch is discarded.

If all the batches are discarded, then the individual peptide with the highest hydropathy score is removed (placed in the OUT list) and preferably replaced by the first peptide, if any, from the second subset (EXTRA list). If the EXTRA list is empty, the generation of batches is performed again from a TOP list with N-1 peptides. Step (b) is then repeated.

If at least one batch is satisfactory, the distribution with the lowest range of hydropathy scores between the different expression cassettes is chosen. By "range" (R) it is meant the highest gap between hydropathy scores of at least two fusion peptides, i.e. (HSK7)ₘₐₓ - (HSK7)ₘᵢₙ. Note, if the ranges are really low (for instance below a range threshold (RThresh) as depicted by **Figure 4**, usually set to 0.005) several different batches with such "low range" can be considered, i.e. there is more than one optimal distribution of the candidate peptides per cassette. In a preferred embodiment, the process of the invention comprises determining an optimal distribution of the candidate peptides from said first subset to the expression cassette(s) among a plurality of possible distribution, presents the lowest range between the hydropathy scores of at least two fusion peptides.

### Step (c) of the designing process: intra-cassette slot assignment

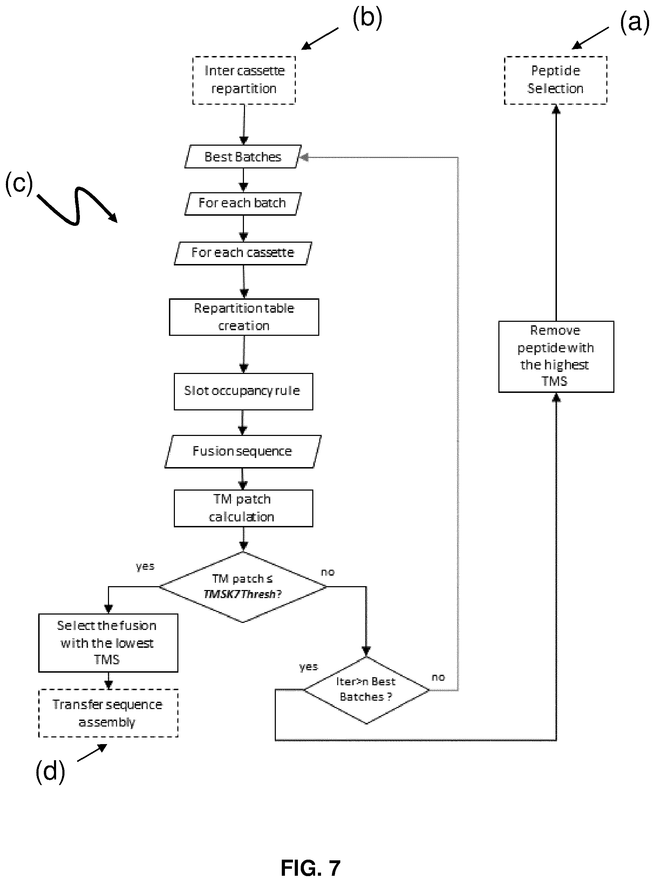

In a step (c) which is detailed by **Figure 7**, the processing means 11 determines for each expression cassette an optimal slot assignment of the candidate peptides as function of cassette slot occupancy rule, wherein said candidate peptides are distributed to the expression cassette as described in step (b). By" slot assignment" of the candidate peptides within a cassette, it is meant an order of the peptides in the expression cassette. In other words, for each "slot" of the cassette, a peptide is chosen among the ones distributed to this cassette.

By "optimal" slot assignment, it is meant, again, the assignment having the lowest global risk of not generating or producing any recombinant poxvirus, criteria for reaching such optimal slot assignment will be explained below.

The risk to generate transmembrane domains is evaluated by generating, for each cassette, all possible peptides combinations with the slot occupancy rule. It has been indeed observed that TM segments may be generated by the junction of 2 particular peptides (inter peptide TM). In this case, modifying the order of peptides in the fusion is an option to eliminate the presence of TM. For instance, if a TM segment results from the fusion of peptide 1 at the N-terminus of peptide 2, inverting the peptides (fusion of peptide 2 at the N-terminus of peptide 1) may eliminate the risk of having a TM segment. Thus, this step performs all the combinations of individual peptides to form a fusion protein.

In a preferred embodiment, step (c) determines an optimal slot assignment of the candidate peptides for each expression cassette based on a cassette slot occupancy rule defining possible slot positions of peptides within a cassette according to transmembrane scores of the peptides, and, in case of equal transmembrane scores, according to their hydropathy scores. More preferably, the candidate peptides distributed to an expression cassette are classified according to at least three classes of risk of not generating or producing any recombinant poxvirus, wherein said cassette slot occupancy rule defines, for each slot positions of a cassette, what class a candidate peptide shall have to be assigned to this slot position.

By "slot occupancy rule", it is meant a rule defining slot positions of peptides within a cassette according to the peptides classes "Low" (L), "Medium" (M) or "High" (H), based on transmembrane and hydropathy scores as defined in step (b).

The "cassette slot occupancy rule" is a clever way to reduce the number of possible combinations. Indeed, for a cassette of 10 peptides, there are 10!, i.e. more than 3.6 million, possible intra-cassette slot assignments.

The idea is to select slots according to these low, medium or high classes, so as to limit the number of possible combinations while minimizing the total TM score. Indeed, each peptide has only a reduced number of possible slots, reducing considerably the number of possible combinations.

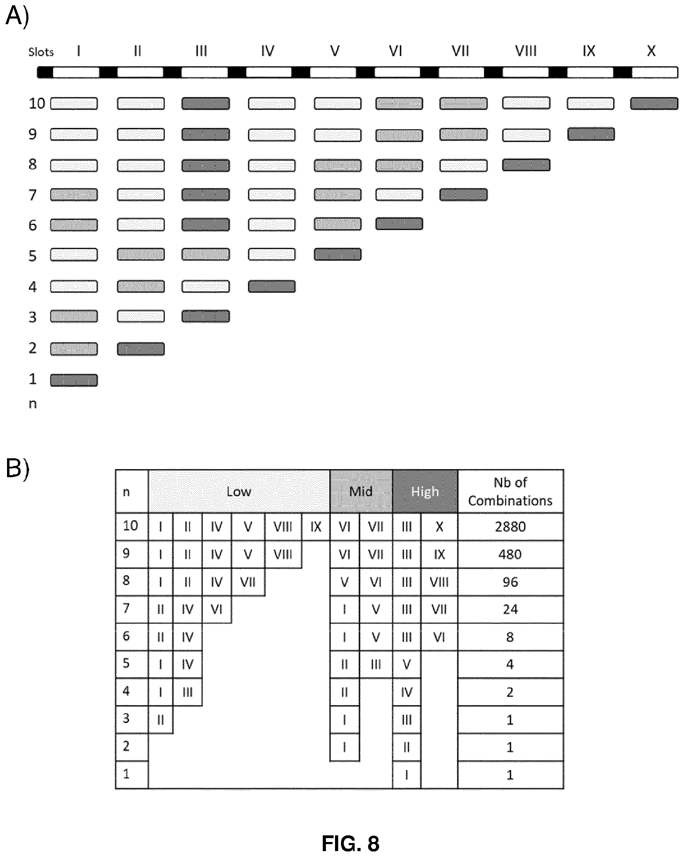

**Figures 8A and 8B** are two representations of a possibility of slot occupation rule. For example, **Figure 8A** shows that the H peptides are preferably placed at the end of the fusion (wherein there is no risk of creating an intra-cassette TM) and in third slot (away from the other H peptide).

To follow the N=13 example developed in step (b), **Figure 8B** shows that in the first cassette (7 peptides), the 3 low class peptides shall be placed in slots II, IV and VI, the 2 medium class peptides shall be placed in slots I and V, and the 2 high class peptides shall be placed in slots III and VII ; and in the second cassette (6 peptides), the 2 low class peptides shall be placed in slots II and IV, the 2 medium class peptides shall be placed in slots I and V, and the 2 high class peptides shall be placed in slots III and VI. Note that there are only 3!x2!x2!=24 possible combinations for the first cassette (instead of 7!=5040) and 2!x2!x2!=8 possible combinations for the second cassette (instead of 6!=720). Even for the worst case of 10 peptides in the cassette (first line) there are only 6!x2!x2!=2880 possible combinations instead of 3.6 million.

In a preferred embodiment, each combination generated in step (c) are evaluated for their risk to generate TM domains which would impair the generation and production of a recombinant virus. More particularly, this step comprises calculating the TM patches for each fusion peptide, comparing said TM patches with a threshold and discarding any fusion that displays at least one TM patch with a score above said threshold. Even more particularly, this step comprises calculating the TM patches for each fusion peptide, comparing said TM patches with the fusion peptide TMS threshold (TMSK7) and discarding any fusion peptide that displays at least one patch with a score above the TMSK7. Preferably, said TM patch is equal to or below a fusion TMS threshold of 60, more preferably a threshold of 50, with a specific preference for a threshold of 40 (e.g. of preferred TM patches: 40, 39, 38, 37, 36, 35, 34, 33, 32, 31, 30, etc.).

If all fusion peptides are discarded, then the intra-cassette slot assignment must be performed with the next batch of peptides that passed the inter-cassette distribution step. In the case where no other batch is available, similarly to what is proposed for step (b), the peptide with the highest individual transmembrane score is advantageously removed from the TOP list and replaced by the first one from the EXTRA list. The new TOP list is then reprocessed at the previous step (b). In the case where the EXTRA list is empty, the TOP list is used with the remained elements (N-1).

If all fusion peptides satisfy the TMSK7 threshold, the global TMS is computed and the best combination for each fusion peptide is then ranked based on said global TMS. In a preferred embodiment, step (c) comprises the selection of the fusion peptide with the lowest TM score.

### Step (d) of the designing process: back translation

In one embodiment, the process of the present invention further comprises a so-called "back translation" step (d) of determining a DNA (nucleic acid molecule) transfer sequence comprising at least one of the expression cassette(s) to be inserted in the recombinant poxvirus (**Figure 9**). Preferably, the DNA transfer sequence may also include elements to ease generation of the recombinant poxvirus (e.g.: restriction sites and/or recombination arm).

This function back-translates (from amino acid to nucleotide sequence) each expression cassette that encodes a codon-optimized fusion protein and inserts them into a predefined DNA backbone required to produce a plasmid (i.e. a transfer plasmid) that will be used to generate the recombinant poxvirus. This final DNA sequence is called the "Transfer Sequence".

The output (in particular on the interface (13)) is advantageously a unique transfer sequence written into a file, for instance in FASTA format, to be sent to a plasmid synthesis external provider. Such step (d) of generating a transfer sequence is well known to the skilled person and will not be discussed in detail.

The back translation step (d) may be customized and optimized so as to provide optimal DNA sequences about various features useful for expression of the one or more peptide fusion(s), and taking into account intermediate cloning steps, insertion site(s) within the recombinant poxvirus genome and the generation of the recombinant poxvirus (design ruled by the plasmidFeatures.yml configuration file).

In one embodiment, each peptide fusion is backtranslated into DNA according to the "most frequent" codon usage and grafted in the draft of the DNA transfer sequence at its expected insertion site. A file in JSON format is then written after the backtranslation. It contains the non-optimized transfer sequence, the coordinates of the regions that must be optimized and the information form the "custMots" section of the plasmiFeatures.yml configuration file. A codon optimization step is then performed for each expression cassette (e.g. using the GeneOptimizer™ tool; ThermoFisher). The optimized sequences are then imported back to the working directories and re-grafted into the transfer sequence backbone. This step is performed with the TSAssembler.sh script that will run the TransferSeqAssembler function for a given PMY ID.

In addition to the optimized codon features, this file also contains information for the sequence optimization process under the "custMots" (custom Motives) section. Optimization can also be carried out by suppressing clusters of rare, non-optimal codons being present in concentrated areas and/or "negative" sequence elements which are expected to negatively influence expression levels. Such negative sequence elements include without limitation the regions having very high (>80%) or very low (<30%) GC content; AT-rich or GC-rich sequence stretches; unstable direct or inverted repeat sequences; internal cryptic regulatory elements (such as internal TATA-boxes, chi-sites, ribosome entry sites, and/or splicing donor/acceptor sites) and/or 5TNT sequences (TTTTT{N}T).

In another embodiment, the file also incorporates the regulatory elements appropriate for optimal expression of the peptide fusion(s), in particular a promoter and a polyA to generate functional expression cassette(s) as described herein.

In another embodiment, the file may also incorporate in the DNA transfer sequence additional elements useful for subsequent cloning of such DNA transfer and generation of the recombinant poxvirus described herein. Desirably, such additional elements include appropriate restriction sites to allow subsequent cloning steps. Desirably, such restriction sites are located 5' and 3' of each expression cassette and/or of the DNA transfer sequence. Preferably, such restriction sites are preferably not present within the peptide fusion-encoding nucleic acid sequence.

In a further embodiment, the file may also incorporate in the DNA transfer sequence the recombination arms, adapted to the selected insertion site in the poxviral genome. Preferably, two recombination arms corresponding to stretches of poxviral sequences homologous (e.g. 90-100% identical) to those present in the parental genome on both sides of the insertion site are incorporated in the DNA transfer sequence 5' and 3' of the one or more expression cassette(s). The length of the recombination arms may vary. Desirably, each of the recombination arms comprises at least 150bp, preferably at least 200bp, more preferably, at least 300bp, even more preferably from 300 to 600 bp with a specific preference for 350 to 500bp (e.g. approximately 350bp or 500bp) or for 300 to 400 bp of homologous poxvirus sequences.

In a preferred embodiment of the invention, the process for designing a recombinant poxvirus, said recombinant poxvirus comprising one or more expression cassettes, each for expression of a fusion of one or a plurality of peptides, comprises performing the steps of:
(a) selecting a first subset of candidate peptides, wherein said peptides present transmembrane scores below a TMS threshold;
(b) determining an optimal distribution of the candidate peptides from said first subset to the expression cassette(s) among a plurality of possible distributions;
(c) for each expression cassette, determining an optimal slot assignment of the candidate peptides as function of cassette slot occupancy rule;
(d) determining a DNA transfer sequence comprising the nucleotide sequence of the one or more expression cassette(s) for generation of said recombinant poxvirus.

### Generation of the recombinant poxvirus

In a second aspect, the invention also describes a process for preparing the recombinant poxvirus described herein. This second process comprises, performing the process according to the first aspect for designing said recombinant poxvirus, and then generating said recombinant poxvirus (as designed).

Typically, such a step of generating the recombinant poxvirus comprises the generation of a DNA transfer plasmid comprising the DNA transfer sequence obtained at step (d) of the first process and the generation of the peptide-expressing recombinant poxvirus, possibly with the manufacturing of said recombinant poxvirus.

The general conditions for constructing recombinant poxviruses are well known in the art (see for example WO2018/234506, WO2007/147528; WO2010/130753; WO03/008533; US 6,998,252; US 5,972,597 and US 6,440,422). Typically, the transfer DNA sequence is cloned in a transfer plasmid and the recombinant poxvirus is generated by homologous recombination between said transfer plasmid comprising the peptide expression cassette(s) flanked in 5' and 3' with the recombination arms adapted to the insertion site within the virus genome. In one embodiment, said process comprises a step of generating said transfer plasmid (e.g. by conventional molecular biology methods) and a step of introducing said transfer plasmid into a suitable host cell, notably together with a poxvirus genome (i.e. a parental virus). Homologous recombination permitting to generate the modified poxvirus is preferably carried out in appropriate host cells (e.g. HeLa or CEF cells). Preferably, the transfer plasmid is linearized before being transfected into the host cell and the parental virus is preferably introduced by infection.

The parental poxvirus may be a wild-type poxvirus or a modified one (e.g. attenuated) as described above in connection with the term "poxvirus". Insertion is then performed by homologous recombination between the stretch of homologous sequences present both in the parental genome and the linearized transfer plasmid, requiring transfection of permissive cells with the linearized transfer plasmid and infection with the parental poxvirus.

The DNA transfer sequence generated at step (d) can independently be inserted at any location of the poxviral genome. Various sites of insertion may be considered, e.g. in a non-essential viral gene, in an intergenic region, or in a non-coding portion of the poxvirus genome. For oncolytic vaccinia virus, J2R locus (encoding TK) is particularly appropriate in the context of the invention and the recombination arms are designed to delete at least partially the J2R locus upon insertion of the DNA transfer sequence into the poxvirus genome, resulting in a TK-defective recombinant poxvirus. Additionally, or independently of TK insertion, one may also consider insertion within the I4L locus (encoding RR) resulting in a RR-defective recombinant poxvirus using appropriate recombination arms. For MVA, deletion III and/or deletion II are particularly adapted for insertion of the one or more expression cassette(s). One may consider in the context of the invention to insert the one or more expression cassette(s) in the same insertion site within the poxviral genome or within different sites (e.g. TK and RR for WR or Copenhagen vaccinia virus, or deletions II and III for MVA).

In certain embodiments, identification of the recombinant poxvirus may be facilitated by the use of a selection and/or a detectable gene. In preferred embodiments, the transfer plasmid further comprises a selection marker with a specific preference for the GPT gene (encoding a guanine phosphoribosyl transferase) permitting growth in a selective medium (e.g. in the presence of mycophenolic acid, xanthine and hypoxanthine).

In certain embodiments, the step of generating the recombinant poxvirus encompasses the use of a parental poxvirus comprising a reporter gene encoding a detectable gene product and, notably, a fluorescent reporter gene, cloned at the site of insertion that is selected for the peptide-expression cassette(s). Preferably, the reporter gene is placed under the transcriptional control of a promoter allowing its expression within the permissive cells, e.g. a vaccinia promoter. This embodiment facilitates the selection of the recombinant poxvirus with respect to the parental poxvirus. Representative examples of fluorescent reporters that can be used in the context of the present invention include, without limitation, GFP (Green Fluorescent Protein), eGFP (Enhanced Green Fluorescent Protein), AmCyan 1 fluorescent protein and mCherry. For instance, when relying on mCherry (a monomeric fluorescent protein that originates from a Discosoma mushroom with peak absorption/emission at 587 nm and 610 nm), the recombinant viruses having inserted the peptide-encoding nucleic acid molecule(s) or expression cassette(s) in place of the mCherry-encoding sequences, will give rise to white plaques whereas the parental viruses retaining the mCherry expression cassette will give rise to red plaques The selection of the recombinant poxvirus may be by direct visualization (white plaques for recombinant poxviruses whereas parental viruses appear in red) or may also be facilitated by sorting means such as FACS after labelling with an APC (Allophycocyanin)-tagged anti-vaccinia virus antibody. A vast number of anti-vaccinia antibodies is available from commercial sources.

The step of generating the recombinant poxvirus may comprise a further step of cleavage by an endonuclease able to generate at least one double strand break in the reporter (e.g. mCherry) nucleotide sequence but in which said endonuclease does not cleave the poxviral genome. Said endonuclease may be in the form of a protein or expressed by an expression vector. The suitable endonuclease is preferably selected from the group consisting of zinc finger nucleases (ZFNs), transcription activator-like effector nucleases (TALENs), clustered regularly interspaced short palindromic repeats (CRISPR)/Cas9 nucleases and restriction enzymes with unique cleavage within the fluorescent reporter gene. The use of the CRISPR/CAS9 system for virus editing is described in the art (Yuan et al., 2015, J. Virol 89, 5176-9; Yuan et al., 2016, Viruses 8, 72, doi:10.3390) requiring the use of a plasmid encoding a Cas9 without a nuclear localization signal as well as a suitable guide RNA. In this consideration, further to infection with the parental virus, the permissive cells may be transfected with the transfer plasmid, the Cas9-expressing plasmid and one or more plasmid(s) encoding the guide RNA (e.g. mCherry-targeted guide RNA).

The selection of recombinant poxviruses is then performed visually (direct isolation of white plaques corresponding in theory to the recombinant poxviruses, whereas colored plaques correspond to the parental; the color depends on the reporter gene) or using conventional sorting means (FACS optionally after a labelling step with appropriate antibodies as described above).

Usually, with conventional techniques without any process of designing, a low percentage of white plaque is obtained (about 1%) and about one recombinant poxvirus is obtained for 50 to 100 parental viruses (about 1% to 2%), whereas the process of the present invention allows to increase the generation of recombinant poxvirus, as reflected by the increase of the presence of white plaques and the increase of the number of recombinant poxviruses. Desirably, said process for generating recombinant poxviruses permits to reach a yield of at least 2%, preferably at least 3%, more preferably at least 4% of white plaques, and at least 50%, preferably at least 60%, more preferably 70% and even more preferably at least 80% of recombinant poxviruses.

Recombinant poxvirus may then be identified by an analysis step of the white plaques thus generated (preferably analysis by PCR) to confirm insertion of expression cassette(s) in the poxviral genome.

### Manufacturing of the recombinant poxvirus

Once generated by the process of the present invention, the recombinant poxvirus may be produced/amplified using conventional techniques. Therefore, the process of the present invention may further, comprise a step of manufacturing said recombinant poxvirus. In a preferred embodiment, said manufacturing step comprises an amplification step in a suitable producer cell to a suitable scale, a step of recovery of the produced recombinant poxvirus from the cell culture and an optional step of purification of the recovered recombinant poxvirus. Such steps are conventional in the art (see e.g. WO2007/147528 or WO2018/234506).

Briefly, the amplification step includes cultivation of a producer (e.g. permissive) host cell, the infection of the cultured producer host cells, and cultivation of the infected host cell under suitable conditions so as to allow the production of the recombinant poxvirus (e.g. infectious viral particles). The choice of the producer cell depends on the type of recombinant poxvirus to be amplified. MVA is strictly host-restricted and is typically amplified on avian cells, either primary avian cells (such as chicken embryo fibroblasts (CEF) prepared from chicken embryos obtained from fertilized eggs) or immortalized avian cell lines, e.g. with a duck TERT gene (see e.g. WO2007/077256, WO2009/004016, WO2010/130756 and WO2012/001075); or obtained from embryonic cells by progressive severance from growth factors and feeder layer (e.g. Eb66 described in Olivier et al., 2010, mAbs 2(4): 405-15). For other vaccinia virus or other poxvirus strains, in addition to avian primary cells (such as CEF) and avian cell lines, many other non-avian cell lines are available for production, including human cell lines such as HeLa (ATCC-CRM-CCL-2™ or ATCC-CCL-2.2™) cells (see e.g. WO2010/130753).

Producer cells are preferably cultured in a medium free of animal- or human-derived products, using a chemically defined medium with no product of animal or human origin. Such media are commercially available (e.g. VP-SFM medium (Invitrogen) is appropriate for culturing CEFs). Producer cells are preferably cultivated at a temperature comprised between 30°C and 38°C (more preferably at around 37°C) for between 1 and 8 days (preferably for 1 to 5 days for CEF and 2 to 7 days for immortalized cells) before infection.

Infection of producer cells by the recombinant poxvirus is made under appropriate conditions (e.g. an appropriate multiplicity of infection (MOI)) to permit productive infection of producer cells. Suitable MOI to be used for amplifying poxviruses are typically between 0.001 and 1 (more preferably about 0.05). Infection step can be performed in a medium which may be the same as or different from the medium used for culturing the producer cells.

The infected producer cells are then cultured under appropriate conditions well known to those skilled in the art until progeny viral vector (e.g. infectious virus particles) is produced. Culture of infected producer cells is also preferably performed in a medium which may be the same as or different from the medium used for culturing of producer cells and/or for infection step, preferably at a temperature between 30°C and 37°C, for 1 to 5 days.

The poxviral particles can be collected from the culture supernatant and/or the producer cells. The cell culture supernatant and the producer cells can be pooled or collected separately. Recovery from producer cells may require a step allowing the disruption of the producer cell membrane to allow the liberation of the virus. Various techniques are available to those skilled in the art, including but not limited to freeze/thaw, hypotonic lysis, sonication, micro fluidization or high-speed homogenization, with a preference for the latter (e.g. using a SILVERSON L4R).

The poxviral particles may then be further purified, using purification steps well known in the art. Various purification steps can be envisaged, including clarification, enzymatic treatment (e.g. endonuclease, protease, etc.), chromatographic and filtration steps. Appropriate methods are described in the art (e.g. WO2007/147528; WO2008/138533, WO2009/100521, WO2010/130753, WO2013/022764).

In a preferred embodiment, said manufacturing step reaches the production of at least 10⁹ pfu, desirably at least 5x10⁹ pfu and preferably approximately 10¹⁰ pfu or more for a recombinant poxvirus to be distributed in suitable doses for testing and treatment of the patient.

### Armed recombinant poxviruses

Certain embodiments of this invention also encompass recombinant poxviruses which, further to the peptides-encoding expression cassette(s), comprise additional therapeutic gene(s) inserted in the viral genome. A vast number of therapeutic genes may be envisaged, especially those encoding polypeptides capable of potentiating anti-tumor efficacy of the virus or reinforcing the host's immunity. Preferred therapeutic genes are selected from the group consisting of suicide genes (genes coding for a protein able to convert a drug precursor into a cytotoxic drug) and immunostimulatory genes (therapeutic genes that encode polypeptides able to stimulate the immune system or effector cells, in a specific or non-specific way).

### Recombinant poxvirus Composition

According to a third aspect, the invention proposes a composition comprising a recombinant poxvirus obtained using the process according to the second aspect of the invention, comprising one or more expression cassettes, each for expression of a fusion of a plurality of peptides (e.g. neopeptides) described herein.

In one embodiment, the composition according to the third aspect of the present invention is in the form of a pharmaceutical composition comprising the recombinant poxvirus described herein (or obtained accordingly to the process described herein) and a pharmaceutically acceptable vehicle. In a preferred embodiment, the composition comprises a therapeutically effective amount of said recombinant poxvirus.

The term "pharmaceutically acceptable vehicle" is intended to include any and all carriers, solvents, diluents, excipients, adjuvants, dispersion media, coatings, antibacterial and antifungal agents, absorption agents and the like compatible with administration in mammals and in particular human subjects.

A "therapeutically effective amount" corresponds to the amount of the recombinant poxvirus required to produce in a subject treated in accordance with the present invention an observable improvement of his clinical status including at least one of those mentioned herein.

Such a therapeutically effective amount may vary depending upon a variety of factors, including but not limited to the characteristics of the poxvirus (including type of virus, bioavailability and doses), the severity and the course of the disease (cancer grade for example), the subject himself (including age, sex, clinical history, general physical condition, etc.), the nature of the pharmaceutically acceptable carrier or excipient in the virus formulation, and the treatment modalities (route of administration, frequency of administration, type of concurrent medication, etc.). The appropriate dosage of poxvirus may be routinely determined and adapted by a practitioner in the light of the relevant circumstances, for example by monitoring a subject's response to administration of the virus and adjusting the dosage accordingly (for additional guidance, see Remington, The Science and Practice of Pharmacy; Gennaro ed., Pharmaceutical Press, London, UK; e.g. 22nd Edition or subsequent ones).

For illustrative purposes, a suitable therapeutically effective amount for individual doses may vary from approximately 10⁵ to approximately 10¹³ vp (viral particles), iu (infectious unit) or pfu (plaque-forming units) depending on the poxvirus and the quantitative technique used. As a general guidance, individual doses from approximately 10⁶ pfu to approximately 10¹¹ pfu are particularly appropriate in the context of the present invention, more preferably from approximately 5x10⁶ pfu to approximately 5x10⁹ pfu; even more preferably doses of approximately 10⁷ pfu to approximately 10⁹pfu, with a preference for individual doses comprising approximately 5x10⁷ or 10⁸ pfu of recombinant poxvirus. Individual doses may be reduced by a factor of 2 to 20 for local administration(s) such as intratumoral injection. The quantity of virus present in a sample can be determined by routine titration techniques, e.g. by counting the number of plaques following infection of permissive cells (e.g. BHK-21 or CEF), immunostaining (e.g. using anti-virus antibodies), by measuring the A260 absorbance (vp titers), by quantitative immunofluorescence (iu titers) or by qPCR using specific viral primers and probes.

Various formulations can be envisaged in the context of the invention, either liquid or freeze-dried to ensure virus stability under the conditions of manufacture and long-term storage (i.e. for at least 6 months) at freezing (e.g. -70°C, -20°C), refrigerated (e.g. 4°C) or ambient (e.g. 20-25°C) temperature. The recombinant poxvirus is advantageously placed in a diluent appropriate for human or animal use. Representative examples of suitable diluent include sterile water, physiological saline (e.g. sodium chloride), Ringer's solution, glucose, trehalose or saccharose solutions, Hank's solution, and other aqueous physiologically balanced salt solutions.

Desirably, the poxvirus composition is suitably buffered for human use. Buffers such as TRIS (tris(hydroxymethyl)methylamine), TRIS-HCl (tris(hydroxymethyl)methylamine-HCl), phosphate buffer (e.g. PBS; mixture of Na₂HPO₄ and KH₂PO₄; mixture of Na₂HPO₄ and NaH₂PO₄), and bicarbonate buffers are particularly appropriate for maintaining a physiological or slightly basic pH (e.g. from approximately pH 7 to approximately pH 9). The buffer (e.g. TRIS-HCl) is preferably present at a concentration of 10 to 50 mM.

It might be beneficial to also include a monovalent salt so as to ensure an appropriate osmotic pressure. Said monovalent salt may notably be selected from NaCl and KCl, preferably said monovalent salt is NaCl, notably in a concentration of 10 to 500 mM.

If needed, the composition may also include a cryoprotectant so as to facilitate storage at low temperature. Suitable cryoprotectants include without limitation sucrose, trehalose, maltose, lactose, mannitol, sorbitol and glycerol, for example at a concentration varying from 0.5 to 20% (weight in g/volume in L, referred to as w/v) and preferably from 5 to 15% (w/v), with a preference for about 10%. The presence of high molecular weight polymers such as dextran or polyvinylpyrrolidone (PVP) is particularly suited for lyophilized formulations to protect the recombinant poxvirus during the vacuum drying and freeze-drying steps (see e.g. WO2014/053571).

For illustrative purpose, buffered formulations including NaCl and/or sugar are particularly adapted to the preservation of poxviruses (e.g. Tris 10 mM pH 8 with saccharose 5 % (W/V), Sodium glutamate 10 mM, and NaCl 50 mM; or phosphate-buffered saline with glycerol (10%) and NaCl) as well as those described in WO20 16/087457.

### Therapeutic uses and method of treatment

The recombinant poxvirus or composition thereof described herein and obtained by the process according to the present invention are particularly suited for use for therapeutic purposes (as a therapeutic vaccine). Such use encompasses prophylaxis and/or therapy purposes. Typically, « prophylaxis » indicates an approach for preventing, inhibiting, reducing the likelihood or delaying the onset of a disease or pathological condition (e.g. an hyperproliferative (cancer) or an infectious disease)) whereas therapy refers to an approach for obtaining beneficial or desired results including clinical results.

The beneficial effects provided by the recombinant poxvirus or composition thereof described herein can be evidenced by an observable improvement of the clinical status over the baseline status or over the expected status if not treated according to the modalities described herein. An improvement of the clinical status can be easily assessed by any relevant clinical measurement typically used by physicians or other skilled healthcare staff. For the purposes of the invention, beneficial or desired clinical results include, but are not limited to, one or more of the following: alleviating one or more symptoms resulting from the disease, diminishing the extent of the disease, stabilizing the disease (e.g. preventing or delaying the disease progression, decreasing tumor size, etc.), preventing or delaying the spread (e.g. metastasis) of the disease, preventing or delaying the recurrence of the disease, decreasing the risk of relapse, providing a remission (partial or total) of the disease, decreasing the severity of the disease, decreasing the dose of one or more other medications required to treat the disease, increasing the quality of life, and/or prolonging survival.

Appropriate measurements are performed routinely in medical laboratories and hospitals available to assess a clinical benefit such as blood tests, analysis of biological fluids and biopsies as well as medical imaging techniques and a large number of kits are available commercially. They can be performed before the administration (baseline) and at various time points during treatment and after cessation of the treatment.

In the context of the invention, the beneficial or desired clinical results can be transient (for one or a couple of months after cessation of administration) or sustained (for several months or years). As the natural course of clinical status which may vary considerably from a subject to another, it is not required that the therapeutic benefit be observed in each subject treated but in a significant number of subjects (e.g. statistically significant differences between two groups can be determined by any statistical test known in the art, such as a Tukey parametric test, the Kruskal-Wallis test the U test according to Mann and Whitney, the Student's t-test, the Wilcoxon test, etc.).

In a preferred embodiment, the recombinant poxvirus or composition thereof is for use for the treatment of an hyperproliferative disease. As used herein, the term "hyperproliferative disease" involves abnormal proliferation of cells and spread including cancers and some cardiovascular diseases (e.g. restenosis that results from the proliferation of the smooth muscle cells of the blood vessel wall, etc.). As used herein, the term "cancer" may be used interchangeably with any of the terms "tumor", "malignancy", "neoplasm" and encompasses any disease or pathological condition resulting from uncontrolled cell growth and spread. These terms are meant to include any type of tissue, organ or cell, any stage of malignancy (e.g. from a prelesion to stage IV). Typically, tumors, especially malignant tumors, show partial or complete lack of structural organization and functional coordination as compared to normal tissue and generally show a propensity to invade surrounding tissues (spreading) and/or metastasize to farther sites.

In particular, the present invention provides a recombinant poxvirus or composition thereof for use for treating a cancer or preventing its relapse in a subject. The present invention also relates to a recombinant poxvirus or composition thereof for the manufacture of a medicament for treating a cancer or preventing its relapse. The present invention also relates to a method of treatment comprising administering the recombinant poxvirus obtained using the process according to the second aspect of the invention or a composition thereof in a subject in need thereof in an amount sufficient for treating a cancer or preventing its relapse in said subject.

In a particularly preferred embodiment, the present invention is carried out for designing and generating a recombinant poxvirus or composition for use as a personalized cancer vaccine. The term "personalized" as applied herein refers to either the individual level (a specific subject) or a subpopulation level (a small group of persons sharing a common feature, e.g. having a specific disease, a specific phenotypic feature, or taking the same drug or showing the same deficiency e.g. in the immune system). Particularly appropriate recombinant poxviruses in this context will be designed by the process described herein to comprise one or more expression cassettes, each for expression of a fusion of a plurality of neopeptides as described in the example section.

Representative examples of cancers that may be treated in the context of the present invention include bone cancer, liver cancer, pancreatic cancer, stomach cancer, colon cancer, cancer of the esophagus, oro-pharyngeal cancer, lung cancer, cancer of the head or neck, skin cancer, melanoma, uterine cancer, cervix cancer, ovarian cancer, breast cancer, rectal cancer, cancer of the anal region, prostate cancer, lymphoma, cancer of the endocrine system, cancer of the thyroid gland, sarcoma of soft tissue, chronic or acute leukemias, cancer of the bladder, renal cancer, neoplasm of the central nervous system (CNS), glioma, etc. The present invention is particularly appropriate for the treatment of solid tumors. It is also particularly useful for treatment of advanced cancers, including metastatic solid cancers or cancers associated with high risk of relapse. Preferred cancers for being treated according to the modalities described herein include brain cancer, such as for example astrocytomas, embryonal tumors, germ cell tumors, central nervous system atypical teratoid/rhabdoid tumor, craniopharyngioma, ependymoma, glioma and glioblastoma as well as head and neck cancer. Other type of cancers for being treated according to the modalities described herein are ovarian cancer as well as lung cancers, and particularly NSCLC with a specific preference for adenocarcinoma, squamous cell carcinoma and large cell carcinoma.

For example, the beneficial or desired clinical results may be correlated with one or more of the followings: inhibiting or slowing tumor growth, proliferation and metastasis, preventing or delaying tumor invasion (spread of tumor cells in neighboring tissues), reducing the tumor lesion number; reducing the tumor size, reducing the number or extent of metastases, providing a prolonged overall survival rate (OS), increasing progression free survival (PFS), increasing the length of remission, stabilizing (i.e. not worsening) the state of disease, preventing the recurrence of disease, providing a better response to another treatment, improving quality of life and/or inducing an anti-tumor response (e.g. non-specific (innate) and/or specific such as a cytotoxic T cell response) in the subject treated in accordance with the present invention.

In another embodiment, the recombinant poxvirus or composition thereof is for use for the treatment of infectious diseases. As used herein, the term "infectious disease" refers to a disease resulting from an infection with a pathogenic organism (e.g. bacteria, parasite, virus, fungus, etc.). The present invention also relates to a method of treatment comprising administering the recombinant poxvirus obtained using the process according to the second aspect of the invention or a composition thereof in a subject in need thereof in an amount sufficient for treating an infectious disease or preventing its relapse in said subject.

Representative examples of infectious diseases that may be treated in the context of the present invention include chronic HBV (hepatitis B virus) infection and HPV (human papillomavirus) infection. Particularly appropriate recombinant poxviruses in this context will be designed and generated by the process described herein to comprise one or more expression cassettes, each for expression of a fusion of a plurality of immunogenic peptides obtained from a pathogenic organism. When the method aims at treating an infectious disease, a therapeutic benefit can be evidenced by, for instance, a decrease of the amount of the infecting pathogenic organism quantified in blood, plasma, or sera of a treated subject, and/or a stabilized (not worsening) state of the infectious disease (e.g. stabilization of inflammatory status), and/or the reduction of the level of specific serum markers (e.g. decrease of alanine aminotransferase (ALT) and/or aspartate aminotransferase (AST) associated with liver poor condition usually observed in chronic hepatitis B or C), decrease in the level of any antigen associated with the occurrence of an infectious disease and/or the appearance or the modification of the level of antibodies to the pathogenic organism and/or the release of signals by immune cells (e.g. cytokines) and/or an improved response of the treated subject to conventional therapies (e.g. antibiotics, nucleoside analogs, etc.) and/or a survival extension as compared to expected survival if not receiving the combination treatment.

### Administration of the recombinant poxvirus or composition thereof

Any of the conventional administration routes are applicable with a preference for parenteral routes. Parenteral routes are intended for administration as an injection or infusion and encompass systemic as well as local routes. Particularly suitable administration routes include, without limitation, intravenous (into a vein), intravascular (into a blood vessel), intra-arterial (into an artery), intradermal (into the dermis), subcutaneous (under the skin), intramuscular (into muscle), intraperitoneal (into the peritoneum), intracerebral (into the brain) and intratumoral (into a tumor or its close vicinity) routes as well as scarification. Infusions typically are given by intravenous route or intratumoral (in a large tumor). Mucosal administrations are also contemplated by the present invention and include, without limitation, oral/alimentary, intranasal, intratracheal, nasopharyngeal, intrapulmonary, intravaginal or intra-rectal route. Topical administrations applied directly to the skin or the surface of tissues (e.g. eye drops, ear drops, etc.). Inhalation may also be envisaged especially when the tumor to be treated is in the respiratory tract and lungs. The recombinant poxvirus or composition thereof is preferably administered to the patient by intravenous, subcutaneous, intramuscular or intratumoral injections.

Administrations may use standard needles and syringes or any device available in the art capable of facilitating or improving delivery including for example catheters, electric syringe, Quadrafuse injection needles, needle-free injection devices (e.g. Biojector TM device), infusion pumps, sprays, etc. Electroporation may also be implemented to facilitate intramuscular administration. Topical administration can also be performed using transdermal means (e.g. patch, microneedles and the like).

The recombinant poxvirus can be administered in a single dose or more desirably in multiple doses over an extended period of time. In the context of the present invention, it is possible to proceed via sequential cycles of administrations that are repeated after a rest period. Intervals between each poxvirus administration can be from several hours to 6 months (e.g. 24h, 48h, 72h, 1 week, 2 weeks, 3 weeks, 1 month, 2 months, etc.). Intervals can also be regular or not (e.g. 1 administration per week for 6 administrations, then 1 administration every 3 weeks for 1 to 14 administrations). The doses may vary for each administration within the range described above. For illustrative purpose, a preferred therapeutic scheme involves one to 40 (e.g. 2, 3, 4, 5, 6, 7, 8, 9, 10, 15, 20, 25, 30, 35, 40) administrations of 5x10⁶ to 5x10⁹ pfu of a recombinant MVA at approximately 1 or 3 weeks interval until a clinical benefit is observed and every 1 to 6 months after.

### Combination therapies

In further embodiments of the method and therapeutic uses described herein, the recombinant poxvirus or composition thereof may be administered in conjunction with one or more additional anti-cancer therapy/ies which have utility in the treatment of the aforementioned cancers. In particular, the additional anticancer therapy/ies is/are selected from the group consisting of surgery, radiotherapy, chemotherapy, cryotherapy, hormonal therapy, toxin therapy, immunotherapy and cytokine therapy. Such additional anticancer therapy/ies is/are administered to the subject in accordance with standard practice before, after, essentially simultaneously or in an interspersed manner with the recombinant poxvirus or composition thereof.

In specific embodiments, the method or use according to the invention may be carried out in conjunction with surgery. For example, the recombinant poxvirus composition may be administered after partial or total surgical resection of the tumor (e.g. by local application within the excised zone, for example).

In other embodiments, the recombinant poxvirus or composition thereof can be used in association with radiotherapy. Those skilled in the art can readily formulate appropriate radiation therapy protocols and parameters (see for example Perez and Brady, 1992, Principles and Practice of Radiation Oncology, 2nd Ed. JB Lippincott Co; using appropriate adaptations and modifications as will be readily apparent to those skilled in the field). The types of radiation that may be used in cancer treatment are well known in the art and include electron beams, high-energy photons from a linear accelerator or from radioactive sources such as cobalt or cesium, protons, and neutrons. Dosage ranges for radioisotopes vary widely, and depend on the half-life of the isotope, the strength and type of radiation emitted, and the uptake by the neoplastic cells. Regular X-rays doses for prolonged periods of time (3 to 6 weeks), or high single doses are contemplated by the present invention.

In certain embodiments of the invention, the recombinant poxvirus or composition thereof may be used in conjunction with chemotherapy currently available for treating cancer. Representative examples of suitable chemotherapy agents include, without limitation, alkylating agents, topoisomerase I inhibitors, topoisomerase II inhibitors, parp inhibitors, platinum derivatives, inhibitors of tyrosine kinase receptors, cyclophosphamides, antimetabolites, DNA damaging agents and antimitotic agents.

In further embodiments, the recombinant poxvirus or composition thereof may be used in conjunction with immunotherapeutics such as anti-neoplastic antibodies as well as siRNA and antisense polynucleotides. Representative examples include among others monoclonal antibodies blocking specific immune checkpoints such as anti-PD-1, anti-PD-L1 , anti-CTLA-4, anti-LAG3, etc. (e.g. ipilimumab, tremelimumab, pembrolizumab, nivolumab, pidilizumab, AMP-224MEDI4736, MPDL3280A, BMS-936559, etc.), monoclonal antibodies blocking Epidermal Growth Factor Receptor (in particular cetuximab, panitumumab, zalutumumab, nimotuzumab, matuzumab, trastuzumab (Herceptin™), etc.) and monoclonal antibodies blocking Vascular Endothelial Growth Factor (in particular bevacizumab and ranibizumab).

In a preferred embodiment, the administration of the recombinant poxvirus increases survival time in the treated subject as compared to without treatment, e.g. by at least 3 months. Alternatively, it generates a T cell response against said tumor (CD4+ and/or CD8+ T cell response).

The administrations of the recombinant poxvirus composition and the one or more additional anti-cancer therapy may be in intervals, ranging from minutes to weeks. For example, one may provide the subject with the recombinant poxvirus and the additional anti-cancer therapy sequentially or in an interspersed way but concomitant administrations of both therapies within the same period of time are also contemplated. The course of treatment may be routinely determined by a practitioner and various protocols are encompassed by the present invention. For example, 1 to 10 administrations of the recombinant poxvirus composition may be carried out after surgery and chimio/radiotherapy. Moreover, after a course of treatment, it is contemplated that there is a period of time during which no anti-cancer treatment is administered before repetition of treatment cycle(s).

### Examples

### Generation of recombinant MVA

The MVA transfer plasmid is designed to permit insertion of the nucleotide sequence to be transferred by homologous recombination in deletion III of the MVA genome. It contains the Transfer Sequence inserted between the flanking sequences (BRG3 and BRD3) surrounding the MVA deletion III.

The homologous recombination was performed using a parental MVA containing gene encoding for the mCherry fluorescent protein into its deletion III (MVA mCherry). The advantage of MVA mCherry is to differentiate cells that are infected by the recombinant virus which have successfully integrated the expression cassette from the ones that are infected by the initial starting MVA mCherry virus (parental virus). Indeed, mCherry gene is removed in case of successful recombination of the expression cassette within deletion III and the viral plaques appear as white.

Although recombination is a relatively frequent event in vaccinia virus, only 1 to 5% of the recombinant plaques contain the inserted DNA. Therefore, in order to increase the efficacy of homologous recombination, a further step of cleavage by an endonuclease was added. For example, an endonuclease may be used to specifically generate double-strand breaks in the mCherry gene in MVA, and, thus, to increase the efficiency of selection of recombinant MVA (e.g. usually 5 to 50 % of the viral plaques contain the expression cassette).

Generation of MVA was performed by homologous recombination in primary chicken embryos fibroblasts (CEF), as described in patent WO2018/234506. The presence of the expression cassette and absence of contamination by parental MVA was verified by PCR.

### 1. Manual processing of neopeptide repartition

The aim of this experiment was to evaluate the design of a recombinant MVA vaccine expressing up to 30 neopeptides as three fusion proteins.

### a. Material and methods

### Dataset

A list of 30 human neopeptides (29mers) were generated from 30 somatic mutations selected from the public database COSMIC (Catalogue Of Somatic Mutations In Cancer, Tate et al., 2019, Nucleic Acids Res., 47(D1): D941-D947).).

**Table 2. List of the 30 somatic mutations selected from COSMIC**

| **Gene Name** | **Genomic position** | **Mutation** |
|---|---|---|
| **ABCB1** | 7:87504292 | C/A |
| **ACE2** | X:15591766 | C/A |
| **ACTRT1** | 6:56606611 | C/G |
| **ANK1** | 1:214630626 | G/C |
| **BTAF1** | X:128051961 | C/A |
| **DSG3** | 8:41692762 | C/T |
| **DST** | 2:179172218 | C/T |
| **ERAP1** | 5:96795133 | T/C |
| **EXT1** | 8:118110457 | G/A |
| **GALNTL6** | 14:106038048 | A/T |
| **GBE1** | 19:42871887 | C/T |
| **HLA-DQA2** | 6:32746348 | A/G |
| **IARS** | 9:92251878 | T/G |
| **IGHV2-5** | 11:92844054 | G/A |
| **KMT2C** | 7:152235831 | C/G |
| **MED7** | 5:157138785 | A/G |
| **MUC16** | 7:142469851 | G/T |
| **NEB** | 2:151485795 | C/T |
| **OR13C2** | 7:64068972 | T/C |
| **RB1CC1** | 8:52628070 | T/A |
| **SBDS** | 7:66994325 | C/T |
| **SCN9A** | 2:166199718 | G/A |
| **SRGAP1** | 12:64043556 | A/G |
| **TAS2R43** | 12:11091884 | A/G |
| **TMEM144** | 4:158237548 | C/G |
| **TNIK** | 3:171071263 | G/A |
| **TRBV6-6** | 19:8888782 | G/T |
| **ZNF354A** | X:89922404 | C/T |
| **ZNF727** | 9:104605318 | C/T |
| **ZPBP** | 7:50031173 | T/A |

### Software

The prediction of transmembrane domains (TM) and the calculation of the Hydropathy Score of the product of each expression cassette (HSK7) have been performed with the Geneious software (vers 8.1.9, Biomatters Inc).

### b. Results

### Construct with 30 neopeptides: pTG19247

A plasmid construct pTG19247 was generated containing three expression cassettes each encoding ten neopeptides (**Figure 10**). The pool of 30 neopeptides was selected based on their predicted immunogenicity from publicly available patient dataset (study PRJEB3132, Exome sequencing of human lung adenocarcinoma samples and their normal counterparts). The repartition has been made by alphabetical order, without any linker between each peptide. Each cassette comprises a signal sequence (obtained from rabies and measles F glycoproteins) and placed under the control of pH5R, pB2R and p11k7.5 promoters, respectively. To facilitate the construction of the fusions, no linkers were included between the selected neopeptides. Generation of the corresponding virus was performed in CEF by homologous recombination.

During the generation of recombinant MVA using the pTG19247, a low ratio of white plaques (about 1%) was obtained. Only 5 plaques were selected and cloned and PCR analyses revealed that none corresponded to the recombinant viruses (the white plaques result from the presence of parental viruses which have artifactually lost the signal of the mCherry reporter gene) (**Figure 10**).

The analysis of the content of the three fusion proteins revealed the presence of transmembrane domains in the fusion proteins (2 for the expression cassettes B and 3 for C), and a relatively high Hydropathy Score (HSK7>0.5) for expression cassette C. To evaluate the impact of these biochemical properties, 12 peptides involved in TM domains and/or in a high HSK7 were removed from the dataset, leading to a remaining set of 18 neopeptides.

### Construct with 18 neopeptides: pTG19266

The construct pTG19266, has been built based on the 18 "TM free" neopeptides coming from pTG19247. The peptides were divided into three expression cassettes, each coding for a fusion protein of 6 neopeptides to balance the load of each expression cassette. In each fusion, the peptides were also separated by linkers composed by short sequences (5mers) of G and/or T and/or S residues (e.g. GSTSG, GSGSG, etc.) to limit interactions between neopeptides within the fusion protein.

The sequence of fusion peptides designed with the new combinations displayed, as expected, no TM domain, and a reduced HSK7 with a maximum around 0.2 for expression cassette C (**Figure 10**).

Recombinant MVAs were generated using the pTG19266 plasmid, and despite a relatively low percentage of white plaques (1.8%), 6 clones (out of 6) analyzed by PCR were confirmed as true recombinants.

### c. Conclusion

This experiment was designed to evaluate the conditions required to generate a recombinant MVA vaccine which bears several neopeptides as fusion proteins. The absence of transmembrane (TM) domains and a moderate hydropathy score (≤0.2) seemed to be required to generate recombinant MVAs bearing up to 18 neopeptides. The next steps would be to automate the process of neopeptide selection and repartition in accordance to the TM and HSK7 criteria.

### 2. Transfer sequence design with VacDesignR

### a. Material and methods

### Dataset:

Three lists of neopeptides were identified from three Non-Small Cell Lung Cancer samples (P2918, V1035 and V1055) and their normal counterpart (blood). The mutations have been identified by the sequencing laboratory by comparing the exomes from the tumour and the exome from the normal tissue. The ranking was performed by Oncoimmunity based on their expertise.

### VacDesignR parameters

The default parameters were used to run VacDesignR on this dataset, among them:
- Peptide TM score threshold (TMSThresh): 25
- Fusion TM score threshold (TMSK7Thresh): 40
- Fusion hydropathy score threshold (HSK7Thresh): 0.2
- maximum number of peptides (Nmax) per vaccine: 30
- minimum number of peptides (Nmin) per vaccine: 5

### b. Results from VacDesignR

The result from VacDesignR is a map of the positions of the neopeptides within the Transfer Sequence backbone that includes expression cassettes, regulatory elements, terminators and cloning restriction sites. Only relative position of the neopeptides in the expression cassettes are displayed in Table 8 for the three samples. Means of hydrophobicity and predicted number of TM in each fusion protein are also displayed as well as recombinant virus production results, as a percentage of white cells (plaques) and the absolute number of clones of recombinant virus confirmed by PCR.

The following sections will only describe the intermediate results for sample V1055 as more than 30 neopeptides were available as input data. The two other samples had an initial list with less than 30 neopeptides, which led to Transfer Sequence with a lower amount of neopeptides, but similar steps were used for every samples.

### Peptides selection:

The first step consists in the calculation of the TM (transmembrane) score and the TM patches by the tmCalc function of VacDesignR. This step is performed on the entire input dataset as shown in Table 3. The TM score is the sum of the local TM regions reported in TMpatches (eg, the TMpatches '36_1' reports two regions with local TM scores of 36 and 1 respectively. The TM score for this peptide is 36+1=37).

**Table 3. Ranked list of neopeptides from sample V1055 after the TMScore and TMpatches calculation by VacDesignR. The ranking is based on the predicted immunogenicity given by the prediction provider.**

| Rank | PepName | NeoPeptide | Length | TMscore | TMpatches |
|---|---|---|---|---|---|
| 1 | BAIAP3_ENST00000324385_pG1140R | TGVARPQVGGGARARQPVTLHLCRPRAQV | 29 | 0 | 0 |
| 2 | MAGIX_ENST00000376338_pQ130K | LHINGESTQGLTHAKAVERIRAGGPQLHL | 29 | 0 | 0 |
| 3 | SPTA1_ENST00000368147_p.Q2019K | AIEERYAALLKRWEKLLEASAVHRQKLLE | 29 | 0 | 0 |
| 4 | ABCD1_ENST00000218104_p.L93R | LRLLFPRVLCRETGRLALHSAALVSRTFL | 29 | 0 | 0 |
| 5 | MMP15_ENST00000219271_p.G118W | EWMKRPRCGVPDQFWVRVKANLRRRRKRY | 29 | 0 | 0 |
| 6 | TTN_ENST00000342992_p.W3597R | IWQKDGAALSPSPNRRISDAENKHILELS | 29 | 0 | 0 |
| 7 | INTS1_ENST00000389470_p.V872F | ISTLYWKAWPLLLVFAAFNPENIGLAAWE | 29 | 36 | 36 |
| 8 | KDM2B_ENST00000377069_p.G8R | MEAEKDSRRRLRPIDRQRYDEN | 22 | 0 | 0 |
| 9 | MUC16_ENST00000397910_p.P9859H | ITSAVDDTTVFTSNHAFSETRRIPTEPTF | 29 | 0 | 0 |
| 10 | CCDC129_ENST00000319386_p.E731K | HEMEAMKTICQSFRKYLEEIEQHLMGQQA | 29 | 0 | 0 |
| 11 | PIK3CA_ENST00000263967_p.G106V | LRLFQPFLKVIEPVVNREEKILNREIGFA | 29 | 0 | 0 |
| 12 | L1CAM_ENST00000361699_p.Q357H | LYGPGETARLDCQVHGRPQPEVTWRINGI | 29 | 0 | 0 |
| 13 | TENM1_ENST00000371130_p.P413T | TGEVDIGAQVMQTITPGLFWRFQITIHHP | 29 | 1 | 1 |
| 14 | KIAA1211_ENST00000264229_pG846V | DPVMPGGEEKASPFVIKLRRTNYSLRFNC | 29 | 0 | 0 |
| 15 | CD22_ENST00000085219_p.S69W | SFILFHNPEYNKNTWKFDGTRLYESTKDG | 29 | 0 | 0 |
| 16 | AMER1_ENST00000330258_p.G13A | METQKDEAAQAKAAAASGSTREQTAEK | 27 | 0 | 0 |
| 17 | ARHGAP4_ENST00000420383_p.R602W | GRTMSRSWKPRCPHWRMTWRGSWRLWPAL | 29 | 0 | 0 |
| 18 | RBM47_ENST00000295971_p.E123Q | KNRGYAFVMYCHKHQAKRAVRELNNYEIR | 29 | 0 | 0 |
| 19 | NAV3_ENST00000228327_p.K93N | DSKIYTDWANHYLANSGHKRLIKDLQQDI | 29 | 0 | 0 |
| 20 | LARS_ENST00000274562_pM9631 | LGSMPELKKYMKKVIPFVAMIKENLEKMG | 29 | 0 | 0 |
| 21 | ARHGEF40_ENST00000298693_p.S1366F | LQATSPEIKLKWTSFIAQLLWRQAAHNKE | 29 | 0 | 0 |
| 22 | ELP4_ENST00000379163_p.K428N | GSLQHSTFLMSFLANATAFASRLVRHSEP | 29 | 6 | 6 |
| 23 | POM121L2_ENST00000377451_p.D570Y | TSVVMSTTLASTSKYSVFKPPLDFGVVNV | 29 | 0 | 0 |
| 24 | ZNF676_ENST00000397121_p.R1651 | CMLSHLSQHERIYTIENSYKCEENGKAFN | 29 | 0 | 0 |
| 25 | KLHL7_ENST00000322231_p.V455L | ELCPMIEARKNHGLLFVKDKIFAVGGQNG | 29 | 0 | 0 |
| 26 | CEP170B_ENST00000414716_p.P1499A | DLLTGNRSLASSAQAGLGKGRVAAQSPPS | 29 | 0 | 0 |
| 27 | ADORA1_ENST00000309502_p.R108Q | SILALLAIAVDRYLQVKIPLRYKMVVTPR | 29 | 1 | 1 |
| 28 | STIM2_ENST00000237364_p.P821 L | IPHDLCHNGEKSKKLSKIKSLFKKKSK | 27 | 0 | 0 |
| 29 | CACNA1F_ENST00000323022_p.D1040Y | PQECKGSFLVYPDGYVSRPLVRERLWVNS | 29 | 0 | 0 |
| 30 | CRB1_ENST00000543483_p.T282S | FVEAVSKTLKLIGISLPWRTSRLAHH | 26 | 1 | 1 |
| 31 | ADORA1_ENST00000367235_p.R108Q | SILALLAIAVDRYLQVKIPLRRISQCMAS | 29 | 1 | 1 |
| 32 | TLL1_ENST00000507499_p.L527F | ENPGIASKARESLIFIWKPIERHDNCAYD | 29 | 0 | 0 |
| 33 | SLC2A13_ENST00000280871_p.R421L | ILALGFVLSAQVSPLITFKPIAPSGQNAT | 29 | 16 | 16 |
| 34 | DEF6_ENST00000316637_p.W237C | LWKRGHLRRNWAERCFQLQPSCLCYFGSE | 29 | 0 | 0 |
| 35 | DUS3L_ENST00000309061_p.T314M | RLDIRGKLYLAPLTMCGNLPFRRICKRFG | 29 | 0 | 0 |
| 36 | ITM2A_ENST00000373298_p.R229L | IYQLCNNRKSFRLRLRDLLLGFNKRAIDK | 29 | 0 | 0 |
| 37 | EFNA4_ENST00000359751_p.E110V | RWVCSLPFGHVQFSVKIQRFTPFSLGFEF | 29 | 0 | 0 |
| 38 | SPEG_ENST00000312358_p.L3199V | PNTSQSATLFLRKVVSVHPWSRPSLQDCL | 29 | 0 | 0 |
| 39 | SCRN2_ENST00000290216_p.S202L | ARNISNQLSIGTDILAQHPELRTHAQAKG | 29 | 0 | 0 |
| 40 | ZSWIM5_ENST00000359600_p.E557D | CARVDALRSHGYPKDALRLTVAIINTLRL | 29 | 0 | 0 |
| 41 | ADAMTS17_ENST00000268070_p.W417C | HSSCAGRSHIMSGECVKGRNPSDLSWSSC | 29 | 0 | 0 |
| 42 | IQSEC3_ENST00000326261_pG310C | DLKNKQIEMLEHKYCGHLVSRRAACTIQT | 29 | 0 | 0 |
| 43 | PPFIA2_ENST00000333447_p.T710S | ASSSPPSGHSTPKLSPRSPAREMDRMGVM | 29 | 0 | 0 |
| 44 | DYSF_ENST00000258104_p.F2037L | TSFLWFTSPYKTMKLILWRRFRWAIILFI | 29 | 0 | 0 |
| 45 | ARSG_ENST00000448504_p.Y432C | GALQTVRLERYKAFCITGGARACDGSTGP | 29 | 0 | 0 |
| 46 | CDH10_ENST00000264463_p.S21Y | LLLFLFWVCLPHFCYPEIMFRRTPVPQQR | 29 | 21 | 21 |
| 47 | UNC5A_ENST00000261961_pS1651 | DPSLDPNVYITREHILVVRQARLADTANY | 29 | 0 | 0 |
| 48 | F12_ENST00000253496_p.K64R | PFQYHRQLYHKCTHRGRPGPQPWCATTPN | 29 | 0 | 0 |
| 49 | NAV3_ENST00000228327_p1634V | QQHSHPNTATVAPFVYRAHSENEGTALPS | 29 | 0 | 0 |
| 50 | MLK4_ENST00000366623_p.G133W | VAFERLELKELIGAWGFGQVYRATWQGQE | 29 | 3 | 3 |
| 51 | MON2_ENST00000280379_p.V824F | EILWRPLTGHLLEKFCQHPNSRMREWGAE | 29 | 0 | 0 |
| 52 | HS3ST4_ENST00000331351_p.P418L | SAPRCLGKSKGRTHLRIDPDVIHRLRKFY | 29 | 0 | 0 |
| 53 | HUWE1_ENST00000262854_p.E1866D | STTSGVVSGSLGSRDINYILRVLGPAACR | 29 | 0 | 0 |
| 54 | TLR8_ENST00000218032_p.P728L | LRTLLLSHNRISHLLSGFLSEVSSLKHLD | 29 | 0 | 0 |
| 55 | GPM6A_ENST00000280187_p.V84M | DTLDVFTMIDIFKYMIYGIAAAFFVYGIL | 29 | 37 | 1_36 |
| 56 | KLHL15_ENST00000328046_pM474I | RTQVVTNCWENKSKINYARCFHKMISYNG | 29 | 0 | 0 |
| 57 | DMD_ENST00000343523_p.W326C | AVLLQRRLDNMNFKCSELRKKSLNIRSHL | 29 | 0 | 0 |
| 58 | GRM8_ENST00000339582_p.L48F | QEYAHSIRVDGDIIFGGLFPVHAKGERGV | 29 | 21 | 21 |
| 59 | BTK_ENST00000308731_p.H181Q | ILENRNGSLKPGSSQRKTKKPLPPTPEED | 29 | 0 | 0 |
| 60 | PHKA2_ENST00000379942_p.G182R | LVFYIEAAYKVADYRMWERGDKTNQGIPE | 29 | 0 | 0 |
| 61 | NOD2_ENST00000300589_pC608W | GSTAPLEFLHITFQWFFAAFYLALSADVP | 29 | 105 | 105 |
| 62 | ZNF212_ENST00000335870_p.G67W | KKMESQAARLQSLEWRTGTAEKKLADCEK | 29 | 0 | 0 |
| 63 | ADAMTS20_ENST00000389420_p.G537V | TQHVPPADGTDCGPVMHCRHGLCVNKETE | 29 | 0 | 0 |

The neopeptides with a TM score above a threshold (TMScore ≥ 25) are filtered out and sent to the OUT list (Table 4). The reason is also reported in this list, as both Filter_Code and Filter_Reason fields. As an example, a Filter_ Code of "1" means "TMScore>threshold".

**Table 4. Content of the OUT list after the filtering step of VacDesignR for sample V1055.**

| Rank | PepName | NeoPeptide | Length | TMscore | TMpatches | Filter Code | Filter Reason |
|---|---|---|---|---|---|---|---|
| 7 | INTS1_ENST00000389470_p.V872F | ISTLYWKAWPLLLVFAAFNPENIGLAAWE | 29 | 36 | 36 | 1 | TMScore≥25 |
| 55 | GPM6A_ENST00000280187_p.V84M | DTLDVFTMIDIFKYMIYGIAAAFFVYGIL | 29 | 37 | 1_36 | 1 | TMScore≥25 |
| 61 | NOD2_ENST00000300589_p.C608W | GSTAPLEFLHITFQWFFAAFYLALSADVP | 29 | 105 | 105 | 1 | TMScore≥25 |

Because the remaining list of neopeptides is higher than 30, two lists are created as the outcome of the neopeptide selection step: in addition to the OUT list, the subfunction listsCreatoR divides the neopeptides into two lists.

Once the input list is filtered, then the neopeptides with the highest rank are selected to be part of the Transfer Sequence and sent to the TOP list (**Table 5**). In addition, the hydropathy score is calculated for each neopeptide of the TOP list. This score corresponds to the mean of the hydropathy scores of the residues of the peptide.

**Table 5. Content of the TOP list after Neopeptide selection by VacDesignR for sample V1055.**

| Rank | PepName | NeoPeptide | Length | TMscore | TMpatches | Hydropathy Score |
|---|---|---|---|---|---|---|
| 1 | BAIAP3_ENST00000324385_pG1140R | TGVARPQVGGGARARQPVTLHLCRPRAQV | 29 | 0 | 0 | 0.34 |
| 2 | MAGIX_ENST00000376338_pQ130K | LHINGESTQGLTHAKAVERIRAGGPQLHL | 29 | 0 | 0 | 0.4 |
| 3 | SPTA1_ENST00000368147_p.Q2019K | AIEERYAALLKRWEKLLEASAVHRQKLLE | 29 | 0 | 0 | 0.41 |
| 4 | ABCD1_ENST00000218104_p.L93R | LRLLFPRVLCRETGRLALHSAALVSRTFL | 29 | 0 | 0 | 0.62 |
| 5 | MMP15_ENST00000219271_p.G118W | EWMKRPRCGVPDQFWVRVKANLRRRRKRY | 29 | 0 | 0 | -1.48 |
| 6 | TTN_ENST00000342992_p.W3597R | IWQKDGAALSPSPNRRISDAENKHILELS | 29 | 0 | 0 | 0.76 |
| 8 | KDM2B_ENST00000377069_p.G8R | MEAEKDSRRRLRPIDRQRYDEN | 22 | 0 | 0 | -2.3 |
| 9 | MUC16_ENST00000397910_p.P9859H | ITSAVDDTTVFTSNHAFSETRRIPTEPTF | 29 | 0 | 0 | 0.37 |
| 10 | CCDC129_ENST00000319386_p.E731K | HEMEAMKTICQSFRKYLEEIEQHLMGQQA | 29 | 0 | 0 | 0.77 |
| 11 | PIK3CA_ENST00000263967_p.G106V | LRLFQPFLKVIEPVVNREEKILNREIGFA | 29 | 0 | 0 | 0.07 |
| 12 | L1CAM_ENST00000361699_p.Q357H | LYGPGETARLDCQVHGRPQPEVTWRINGI | 29 | 0 | 0 | 0.63 |
| 13 | TENM1_ENST00000371130_p.P413T | TGEVDIGAQVMQTITPGLFWRFQITIHHP | 29 | 1 | 1 | 0.1 |
| 14 | KIAA1211_ENST00000264229_pG846V | DPVMPGGEEKASPFVIKLRRTNYSLRFNC | 29 | 0 | 0 | 0.54 |
| 15 | CD22_ENST00000085219_p.S69W | SFILFHNPEYNKNTWKFDGTRLYESTKDG | 29 | 0 | 0 | -1.14 |
| 16 | AMER1_ENST00000330258_p.G13A | METQKDEAAQAKAAAASGSTREQTAEK | 27 | 0 | 0 | -1.19 |
| 17 | ARHGAP4_ENST00000420383_p.R602W | GRTMSRSWKPRCPHWRMTWRGSWRLWPAL | 29 | 0 | 0 | -1.11 |
| 18 | RBM47_ENST00000295971_p.E123Q | KNRGYAFVMYCHKHQAKRAVRELNNYEIR | 29 | 0 | 0 | -1.11 |
| 19 | NAV3_ENST00000228327_p.K93N | DSKIYTDWANHYLANSGHKRLIKDLQQDI | 29 | 0 | 0 | 0.98 |
| 20 | LARS_ENST00000274562_pM9631 | LGSMPELKKYMKKVIPFVAMIKENLEKMG | 29 | 0 | 0 | 0.09 |
| 21 | ARHGEF40_ENST00000298693_p.S1366F | LQATSPEIKLKWTSFIAQLLWRQAAHNKE | 29 | 0 | 0 | 0.43 |
| 22 | ELP4_ENST00000379163_p.K428N | GSLQHSTFLMSFLANATAFASRLVRHSEP | 29 | 6 | 6 | 0.12 |
| 23 | POM121L2_ENST00000377451_p.D570Y | TSVVMSTTLASTSKYSVFKPPLDFGVVNV | 29 | 0 | 0 | 0.54 |
| 24 | ZNF676_ENST00000397121_p.R165I | CMLSHLSQHERIYTIENSYKCEENGKAFN | 29 | 0 | 0 | 0.85 |
| 25 | KLHL7_ENST00000322231_p.V455L | ELCPMIEARKNHGLLFVKDKIFAVGGQNG | 29 | 0 | 0 | 0.04 |
| 26 | CEP170B_ENST00000414716_p.P1499A | DLLTGNRSLASSAQAGLGKGRVAAQSPPS | 29 | 0 | 0 | 0.28 |
| 27 | ADORA1_ENST00000309502_p.R108Q | SILALLAIAVDRYLQVKIPLRYKMVVTPR | 29 | 1 | 1 | 0.72 |
| 28 | STIM2_ENST00000237364_p.P821L | IPHDLCHNGEKSKKLSKIKSLFKKKSK | 27 | 0 | 0 | -1.17 |
| 29 | CACNA1F_ENST00000323022_p.D1040Y | PQECKGSFLVYPDGYVSRPLVRERLWVNS | 29 | 0 | 0 | 0.44 |
| 30 | CRB1_ENST00000543483_p.T282S | FVEAVSKTLKLIGISLPWRTSRLAHH | 26 | 1 | 1 | 0.22 |
| 31 | ADORA1_ENST00000367235_p.R108Q | SILALLAIAVDRYLQVKIPLRRISQCMAS | 29 | 1 | 1 | 0.82 |

The remaining candidates are stored in the EXTRA list (**Table 6**) and can be used if needed as a pool of extra candidates in case of neopeptide disqualification during the processing of the first TOP list.

**Table 6. Content of the EXTRA list after the neopeptide selection step of VacDesignR for sample V1055**

| | Rank PepName | NeoPeptide | Length | TMscore | TMpatches |
|---|---|---|---|---|---|
| 32 | TLL1_ENST00000507499_p.L527F | ENPGIASKARESLIFIWKPIERHDNCAYD | 29 | 0 | 0 |
| 33 | SLC2A13_ENST00000280871_p.R421L | ILALGFVLSAQVSPLITFKPIAPSGQNAT | 29 | 16 | 16 |
| 34 | DEF6_ENST00000316637_p.W237C | LWKRGHLRRNWAERCFQLQPSCLCYFGSE | 29 | 0 | 0 |
| 35 | DUS3L_ENST00000309061_p.T314M | RLDIRGKLYLAPLTMCGNLPFRRICKRFG | 29 | 0 | 0 |
| 36 | ITM2A_ENST00000373298_p.R229L | IYQLCNNRKSFRLRLRDLLLGFNKRAIDK | 29 | 0 | 0 |
| 37 | EFNA4_ENST00000359751_p.E110V | RWVCSLPFGHVQFSVKIQRFTPFSLGFEF | 29 | 0 | 0 |
| 38 | SPEG_ENST00000312358_p.L3199V | PNTSQSATLFLRKVVSVHPWSRPSLQDCL | 29 | 0 | 0 |
| 39 | SCRN2_ENST00000290216_p.S202L | ARNISNQLSIGTDILAQHPELRTHAQAKG | 29 | 0 | 0 |
| 40 | ZSWIM5_ENST00000359600_pE557D | CARVDALRSHGYPKDALRLTVAIINTLRL | 29 | 0 | 0 |
| 41 | ADAMTS17_ENST00000268070_p.W417C | HSSCAGRSHIMSGECVKGRNPSDLSWSSC | 29 | 0 | 0 |
| 42 | IQSEC3_ENST00000326261_pG310C | DLKNKQIEMLEHKYCGHLVSRRAACTIQT | 29 | 0 | 0 |
| 43 | PPFIA2_ENST00000333447_p.T710S | ASSSPPSGHSTPKLSPRSPAREMDRMGVM | 29 | 0 | 0 |
| 44 | DYSF_ENST00000258104_p.F2037L | TSFLWFTSPYKTMKLILWRRFRWAIILFI | 29 | 0 | 0 |
| 45 | ARSG_ENST00000448504_p.Y432C | GALQTVRLERYKAFCITGGARACDGSTGP | 29 | 0 | 0 |
| 46 | CDH10_ENST00000264463_pS21Y | LLLFLFWVCLPHFCYPEIMFRRTPVPQQR | 29 | 21 | 21 |
| 47 | UNC5A_ENST00000261961_pS165I | DPSLDPNVYITREHILVVRQARLADTANY | 29 | 0 | 0 |
| 48 | F12_ENST00000253496_p.K64R | PFQYHRQLYHKCTHRGRPGPQPWCATTPN | 29 | 0 | 0 |
| 49 | NAV3_ENST00000228327_p1634V | QQHSHPNTATVAPFVYRAHSENEGTALPS | 29 | 0 | 0 |
| 50 | MLK4_ENST00000366623_p.G133W | VAFERLELKELIGAWGFGQVYRATWQGQE | 29 | 3 | 3 |
| 51 | MON2_ENST00000280379_p.V824F | EILWRPLTGHLLEKFCQHPNSRMREWGAE | 29 | 0 | 0 |
| 52 | HS3ST4_ENST00000331351_p.P418L | SAPRCLGKSKGRTHLRIDPDVIHRLRKFY | 29 | 0 | 0 |
| 53 | HUWE1_ENST00000262854_p.E1866D | STTSGVVSGSLGSRDINYILRVLGPAACR | 29 | 0 | 0 |
| 54 | TLR8_ENST00000218032_p.P728L | LRTLLLSHNRISHLLSGFLSEVSSLKHLD | 29 | 0 | 0 |
| 56 | KLHL15_ENST00000328046_pM474I | RTQVVTNCWENKSKINYARCFHKMISYNG | 29 | 0 | 0 |
| 57 | DMD_ENST00000343523_p.W326C | AVLLQRRLDNMNFKCSELRKKSLNIRSHL | 29 | 0 | 0 |
| 58 | GRM8_ENST00000339582_p.L48F | QEYAHSIRVDGDIIFGGLFPVHAKGERGV | 29 | 21 | 21 |
| 59 | BTK_ENST00000308731_p.H181Q | ILENRNGSLKPGSSQRKTKKPLPPTPEED | 29 | 0 | 0 |
| 60 | PHKA2_ENST00000379942_p.G182R | LVFYIEAAYKVADYRMWERGDKTNQGIPE | 29 | 0 | 0 |
| 62 | ZNF212_ENST00000335870_p.G67W | KKMESQAARLQSLEWRTGTAEKKLADCEK | 29 | 0 | 0 |
| 63 | ADAMTS20_ENST00000389420_p.G537V | TQHVPPADGTDCGPVMHCRHGLCVNKETE | 29 | 0 | 0 |

### Inter-cassette distribution:

The 30 neopeptides that are part of the TOP list were divided into three expression cassettes. The repartition rule predefined in VacDesignR has been used to balance the three fusion proteins in term of hydrophobicity and of risk of creating transmembrane domains between neopeptides. Thus, a relative classification of High (H), Medium (M) and Low (L) risk has been applied to the 30 neopeptides. As respect to the repartition rule, each expression cassette will be composed of ten neopeptides: two high class, two medium class and six low class. The selection of peptides from each class is randomly made 30,000 times to generate 30,000 batches of three expression cassette compositions. Hydrophobicity means are then calculated for each set of neopeptides and for each batch, a range is also calculated. Any batch with at least one cassette hydropathy score (HSK7) above the threshold is discarded. The batch with the lowest range of HSK7 was kept for the next step. In the example of sample V1055, the best batch selected for the intra-cassette slot assignment step had a range of cassette hydropathy scores of 0.028 (**Table7**).

**Table 7. Best batch selected after the inter-cassette distribution step for sample V1055. The Rank column refers to the immunogenicity prediction. The HSK7 value considers the signal peptide and the linkers in the calculation of the score.**

| Rank | PepName | Cassette | HSK7 |
|---|---|---|---|
| 3 | SPTA1_ENST00000368147_p.Q2019K | A | -0.316 |
| 6 | TTN_ENST00000342992_p.W3597R | | |
| 11 | PIK3CA_ENST00000263967_p.G106V | | |
| 15 | CD22_ENST00000085219_p.S69W | | |
| 18 | RBM47_ENST00000295971_p.E123Q | | |
| 20 | LARS_ENST00000274562_pM9631 | | |
| 22 | ELP4_ENST00000379163_p.K428N | | |
| 26 | CEP170B_ENST00000414716_p.P1499A | | |
| 28 | STIM2_ENST00000237364_p.P821L | | |
| 30 | CRB1_ENST00000543483_p.T282S | | |
| 4 | ABCD1_ENST00000218104_pL93R | B | -0.288 |
| 8 | KDM2B_ENST00000377069_p.G8R | | |
| 12 | L1CAM_ENST00000361699_p.Q357H | | |
| 14 | KIAA1211_ENST00000264229_pG846V | | |
| 16 | AMER1_ENST00000330258_p.G13A | | |
| 19 | NAV3_ENST00000228327_p.K93N | | |
| 21 | ARHGEF40_ENST00000298693_p.S1366F | | |
| 23 | POM121L2_ENST00000377451_p.D570Y | | |
| 29 | CACNA1F_ENST00000323022_p.D1040Y | | |
| 31 | ADORA1_ENST00000367235_p.R108Q | | |
| 1 | BAIAP3_ENST00000324385_pG1140R, | C | -0.316 |
| 2 | MAGIX_ENST00000376338_pQ130K | | |
| 5 | MMP15_ENST00000219271_p.G118W | | |
| 9 | MUC16_ENST00000397910_p.P9859H | | |
| 10 | CCDC129_ENST00000319386_p.E731K | | |
| 13 | TENM1_ENST00000371130_p.P413T | | |
| 17 | ARHGAP4_ENST00000420383_p.R602W | | |
| 24 | ZNF676_ENST00000397121_p.R165I | | |
| 25 | KLHL7_ENST00000322231_p.V455L | | |
| 27 | ADORA1_ENST00000309502_p.R108Q | | |
| | | Range | 0.028 |

### Intra-cassette slot assignment

In this step, each expression cassette is built based on the composition of the neopeptides and in accordance to the slot occupancy rule that has been created in VacDesignR. For each expression cassette coding 10 neopeptides, a total of 2,880 fusions are generated. The content in neopeptides of the final constructs for samples P2918, V1047 and V1055 are shown in Table 8. The TMS and TM patches are calculated for each fusion peptide to detect any TM domain that could occur between two contiguous neopeptides. Thus, if a local TM region was detected (TM patch> TMSK7Thresh), then the fusion was rejected.

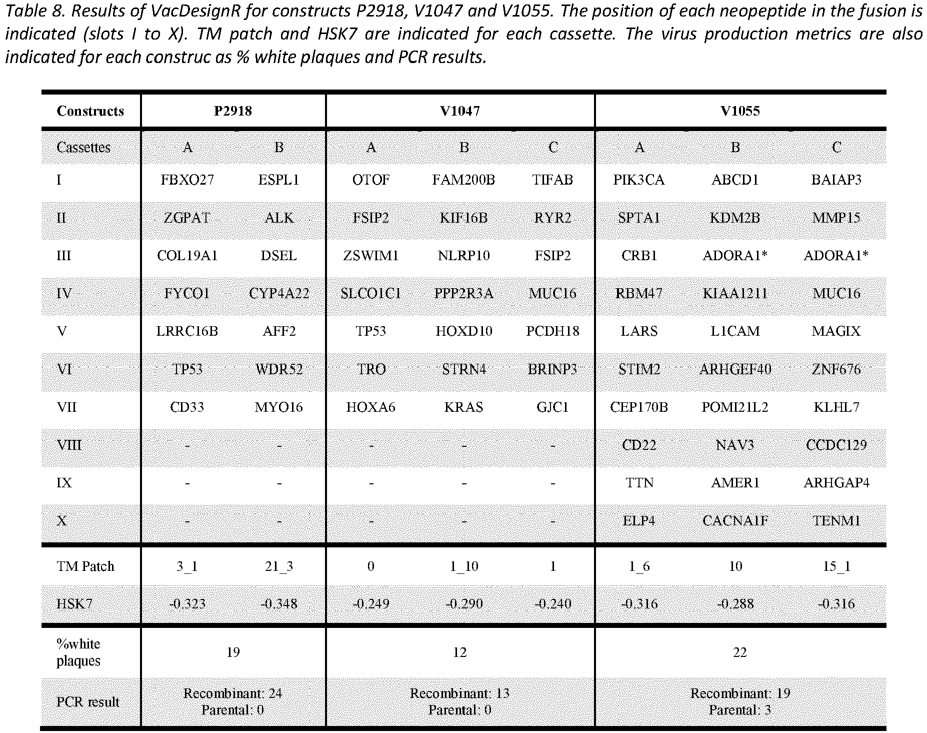

The plasmids were generated for the three samples P2918, V1047 and V1055 coding for respectively 14, 21 and 30 neopeptides in the form of up to three fusion proteins. The final transfer Sequences were in accordance to the previously defined compliance, i.e. no TM domain and a fusion hydropathy Score (HSK7) below 0.2.

The generation of recombinant MVAs with these plasmids was enhanced by the tool. The ranges of % of white plaques was 12 to 22%, and the number of recombinant clones were 13, 19 and 24. The only parental clones still found by PCR were for plasmid from V1055, but with a limited number (3). It has to be noted that only one peptide from the 30 highest ranked in the initial list of neopeptides for sample V1055 was removed due to a TMS issue. It was replaced by the 31^{st} to generate a recombinant MVA virus.

### c. Conclusion

The use of the automated tool, VacDesignR, strongly improved the generation of recombinant MVAs coding for up to 30 neopeptides in three fusion proteins. The improvement of the generation of MVAs was not done at the cost of an over-selection of candidate peptides with a lower interest than the top ranked ones. This improvement was helpful to the design of optimized personalized vaccines.

All of the above cited disclosures of patents, publications and database entries are specifically incorporated herein by reference in their entirety. Other features, objects, and advantages of the invention will be apparent from the description and drawings and from the claims. The following examples are incorporated to demonstrate preferred embodiments of the invention. However, in light of the present disclosure, those skilled in the art should appreciate that changes can be made in the specific embodiments that are disclosed without departing from the spirit and scope of the invention.

### REFERENCES

Antoine et al., 1998, Virol. 244: 365-96
Chakrabarti et al., 1997, Biotechniques 23: 1094-7
Dayhoffed, 1981, Suppl., 3: 482-9
Depla et al., 2008, J. Virol. 82(1): 435-450
Eisenberg et al., 1982, Nature, Sep 23;299(5881):371-4
Eisenberg et al., 1984, Annual review of biochemistry 53.1: 595-623
Erbs et al., 2008, Cancer Gene Ther. 15(1): 18-28)
Guse et al., 2011, Expert Opinion Biol. Ther.11(5):595-608
Hammond et al., 1997, J. Virol Methods 66: 135-8
Kallol et al., 2003, J. Chromat. 1000: 637-55
Krogh et al., 2001, J. Mol. Biol. 305: 567-80
Kumar and Boyle, 1990, Virology 179: 151-8
Kyte and Doolittle, 1982, J. Mol. Biol. 157: 105-32
Mayr et al., 1975, Infection 3: 6-14
Nielsen et al., 2010, Immunology 130(3): 319-28
Olivier et al., 2010, mAbs 2(4): 405-15
Perez and Brady, 1992, Principles and Practice of Radiation Oncology, 2nd Ed. JB Lippincott Co
Plotkin, 2008, Clin Infect Dis.47(3): 401-9
Relman, 2008, J Infect Dis. 198(1): 4-5;
Remington, The Science and Practice of Pharmacy; Gennaro ed., Pharmaceutical Press, London, UK; e.g. 22nd Edition or subsequent ones
Rose et al., 1993, Ann. Rev. Biomol. Struc. 22: 381-415
Sutter and Moss, 1992, Proc. Natl. Acad. Sci. USA 89: 10847-51
Sweet et al., 1983, J. Mol. Biol. 171: 479-88
Tate et al., 2019, Nucleic Acids Res., 47(D1): D941-D947
Yuan et al., 2015, J. Virol 89, 5176-9
Yuan et al., 2016, Viruses 8, 72, doi:10.3390
US 5,972,597
US 6,440,422
US 6,686,152
US 6,998,252
WO03/008533
WO2007/077256
WO2007/147528
WO2008/138533
WO2008/138649
WO2009/004016
WO2009/065546
WO2009/100521
WO2010/130753
WO2012/001075
WO2013/022764
WO2014/053571
WO2016/087457
WO2018/234506

## Claims

1. A process for designing a recombinant poxvirus, said recombinant poxvirus comprising one or more expression cassettes, each for expression of a fusion of one or a plurality of peptides, **characterized in that** it comprises performing the steps of:
(a) selecting a first subset of candidate peptides, wherein said peptides present transmembrane scores below a TMS threshold;
(b) determining an optimal distribution of the candidate peptides from said first subset to the expression cassette(s) among a plurality of possible distributions;
(c) for each expression cassette, determining an optimal slot assignment of the candidate peptides as function of cassette slot occupancy rule;
(d) determining a DNA transfer sequence comprising the nucleotide sequence of the one or more expression cassette(s) for generation of said recombinant poxvirus.

2. The process of claim 1, wherein said peptides of step (a) present continuous regions below a homology threshold.

3. The process of claim 2, wherein step (a) comprises discarding peptides if they present transmembrane scores above a TMS threshold and/or an homologous region with a higher ranked candidate above an homology threshold, and selecting a second subset of the identified set of candidate peptides as candidate peptides neither disclosed nor selected in the first subset.

4. The process of any one of claims 1 to 3, wherein step (b) comprises, if at least one fusion peptide presents an hydropathy score above a given threshold, replacing the candidate peptide from the first subset of candidate peptides presenting the highest hydropathy score by a candidate peptide from the second subset, and proceeding again with a random peptide selection.

5. The process of any one of claims 1 to 4, wherein said optimal distribution of step (b) presents the lowest range between the hydropathy scores of at least two fusion peptides;

6. The process of any one of claims 1 to 5, wherein step (c) further comprises discarding any fusion peptide that displays at least one TM patch with a score above a TMSK7 threshold.

7. The process of any one of claims 1 to 6, wherein step (c) further comprises the selection of the peptide fusion with the lowest TM score.

8. The process of claim 6, wherein step (c) comprises, if at least one transmembrane domain is detected in any possible slot assignment of the candidate peptides in an expression cassette, replacing the candidate peptide from the first subset of candidate peptides presenting the highest transmembrane score, by a candidate peptide from the second subset, and repeating steps (b) then (c).

9. The process of any one of claims 1 to 8, wherein said cassette slot occupancy rule defines possible slot positions of candidate peptides within a cassette according to transmembrane scores of the peptides, and, in case of equal transmembrane scores, according to their hydropathy scores.

10. The process of claim 9, wherein the candidates peptides distributed to an expression cassette are classified according to at least three classes of risk of not generating or producing any recombinant poxvirus, wherein said cassette slot occupancy rule defining, for each slot positions of a cassette, what class a candidate peptide shall have to be assigned to this slot position.

11. The process of any one of claims 1 to 10, wherein said optimal slot assignment of the candidate peptides distributed to an expression cassette presents a transmembrane score below a given threshold.

12. The process of any one of claims 1 to 11, wherein there is/are a single expression cassette if the number of selected candidate peptides is below 10, two expression cassettes if the number of selected candidate peptides is between 10 and 14, and three expression cassettes if the number of selected candidate peptides is between 15 and 30.

13. A process for preparing a therapeutic vaccine comprising a recombinant poxvirus, comprising:
- performing the process of any one of claims 1 to 12 for designing said recombinant poxvirus;
- generating said recombinant poxvirus.

14. The process according to claim 13, wherein said process further comprises a manufacturing step of said recombinant poxvirus, wherein said manufacturing step comprises an amplification step in a suitable producer cell to a suitable scale, a step of recovery of the produced recombinant poxvirus from the cell culture and an optional step of purification of the recovered recombinant poxvirus.

15. The process according to any one of claims 13 and 14, wherein said recombinant poxvirus encodes neopeptides and is for use as a personalized cancer vaccine.

16. The personalized cancer vaccine obtained according to the process of claim 15, wherein said personalized cancer vaccine is a composition comprising a therapeutically effective amount of said recombinant poxvirus and a pharmaceutically acceptable vehicle, preferably for use in a subject in need thereof for treating a cancer or preventing its relapse in a subject.
